# EUROPEAN PATENT APPLICATION

(11) **EP 4 640 185 A1**
(43) Date of publication of application: **29.10.2025**
(21) Application number: 22968850.2
(22) Date of filing: 20.12.2022
(51) Int. Cl.: A61F 2/24

(54) **TRANSCATHETER DELIVERY SYSTEM AND METHOD FOR RELEASING ARTIFICIAL HEART VALVE**

(71) Applicant: Venus MedTech (HangZhou) Inc., Binjiang District Hangzhou Zhejiang 310052 (CN)
(72) Inventor: ZENG, Guirong, Hangzhou, Zhejiang 310052 (CN); ZHANG, Zhifei, Hangzhou, Zhejiang 310052 (CN); MA, Renzheng, Hangzhou, Zhejiang 310052 (CN); ZHENG, Mingrui, Hangzhou, Zhejiang 310052 (CN); MA, Tian, Hangzhou, Zhejiang 310052 (CN); LIU, Weiwei, Hangzhou, Zhejiang 310052 (CN); LIU, Zhaogang, Hangzhou, Zhejiang 310052 (CN)
(74) Representative: KIPA AB
(86) International application number: PCT/CN2022/140367
(87) International publication number: WO 2024/130547

(57) **Abstract**

A transcatheter delivery system, comprising a catheter assembly (2) and a control handle (3) connected to a proximal end of the catheter assembly (2). The control handle (3) comprises a first handle (30) and multiple sets of driving mechanisms (4), wherein one set of driving mechanisms (4) comprises : a driven member (41) slidably mounted on the first handle (30) in an axial direction and used for being connected to a corresponding outer sheath tube (22); and a driving member (42) rotatably mounted on the first handle (30), provided with an internal thread (422), and located on the outer periphery of the driven member (41), wherein the driven member (41) is provided with transmission teeth (412) that match the internal thread (422). The control handle (3) further comprises a prompt clamping tooth (60) connected to the driving member (42) and an elastic member (7) linked to the driven member (41). When the elastic member (7) moves axially along with the driven member (41) to correspond to the position of the prompt clamping tooth (60), the elastic member (7) and the prompt clamping tooth (60) interact with each other to provide a hand feeling response and/or a sound response. The delivery system can achieve a better control effect, thus improving the operation experience of operators such as clinicians.

## Description

### TECHNICAL FIELD

The present application relates to the field of medical devices, and in particular to a transcatheter delivery system and a method for releasing a prosthetic heart valve.

### BACKGROUND

The transcatheter delivery system is configured to perform interventional surgery and implant an interventional device into a predetermined location in the body. The transcatheter delivery system generally includes a control handle configured at the proximal end for manipulation by an operator, and a number of elongated, sliding-nested catheters. The proximal end of the control handle is the control end and is connected to each catheter, while the distal end of the control handle is the working end and can be implanted into the body and complete the delivery, release or retrieval of the interventional device through mutual cooperation. Therefore, the control handle is generally configured with sliding or rotating components for driving the relative movement between the catheters along the axial direction.

In the release process of interventional devices, the operator generally determines the timing of release by repeatedly observing visual signals such as imaging equipment, but due to the short release stroke of the interventional device, it is difficult for the operator to simultaneously take into account both handle release and imaging conditions during the release process. In addition, there may be delays in imaging feedback, which increases the difficulty of control for the operator.

### SUMMARY

In order to solve the above technical problems, the present application discloses a transcatheter delivery system, which has opposite distal and proximal ends with an axial direction extending between the distal and proximal ends. The delivery system includes a catheter assembly and a control handle connected to the proximal end of the catheter assembly. The catheter assembly includes at least two tubes that are slidably sleeved with each other. The control handle includes a first handle and multiple sets of driving mechanisms, each set of driving mechanisms is in transmission connection with a corresponding tube of the catheter assembly; wherein one set of driving mechanisms includes:
a driven member, mounted to the first handle and slidable along the axial direction, the driven member being used to connect to the corresponding tube; and
a driving member, rotatably mounted to the first handle, the driving member having internal threads and being located at an outer periphery of the driven member, and the driven member having one or more transmission teeth that engage with the internal threads.

The control handle further includes one or more indicator members connected to the driving member and an elastic member connected to the driven member.

When the elastic member moves axially with the driven member to the position corresponding to the indicator member, the interaction between the elastic member and the indicator member provides a tactile feedback and/or a sound feedback.

In the following, several alternatives are provided, but merely as further additions or preferences, instead of as further limitations to the above-mentioned technical solution. Without technical or logical contradiction, the alternatives can be combined with the above-mentioned technical solution, individually or in combination.

Optionally, the catheter assembly includes an outer sheath connected to the driven member and an inner shaft assembly slidably fitted in the outer sheath, a radial gap defined between the distal ends of the inner shaft assembly and the outer sheath constitutes a loading zone for accommodating an interventional device, and the inner shaft assembly and the outer sheath are configured to be movable relative to each other, so that the loading zone is covered by or exposed from the outer sheath.

The inner shaft assembly and the outer sheath have a predetermined relative position during the relative movement. In the predetermined relative position, the elastic member and the indicator member interact with each other to produce a response.

Optionally, there are multiple relative positions, which include at least a first relative position and a second relative position adjacent to each other in the axial direction.

The total stroke of the inner shaft assembly relative to the outer sheath is M1; and
the stroke of the outer sheath between the first relative position and the second relative position is M2, and M2 is at least 1/3 of M1.

Optionally, the driving member is a driving sleeve and has a tubular structure, the indicator member is located on the inner side of the driving member, and the driven member is a movable base.

Optionally, in the predetermined relative position, the indicator member produces multiple responses when the rotation amplitude of the driving sleeve is less than or equal to one circle.

Optionally, the indicator member is spaced apart from the movement path of the transmission teeth.

The elastic member and the indicator member engage with each other when aligned, and the deformation of the elastic member at least allows the indicator member to rotate with the driving member and override the elastic member.

Optionally, the first handle at least includes a tubular support body, the support body is provided with a guide groove extending axially, the driven member is slidably fitted in the guide groove, and the driving member is rotatably sleeved over an outer periphery of the support body.

The guide groove extends radially through the support body, and the transmission teeth are arranged on both sides of the driven member and extend out of the guide groove.

Optionally, the driving member is of a single-layer or double-layer structure, and the double-layer structure includes:
a bushing, wherein receiving slots are formed on the side wall of the bushing, the indicator member is located within the corresponding receiving slot, and the internal threads are provided on the inner wall of the bushing, and
an outer housing, fixedly sleeved over the bushing's periphery.

Optionally, the indicator member and the bushing are of separate fixed structures.

Optionally, the outer housing covers the receiving slots.

Optionally, the inner edge of the receiving slot is provided with a limiting step, and the edges of the indicator member are fixedly seated on the limiting step.

Optionally, the receiving slot is a slot-shaped hole, and its shape corresponds to that of the indicator member.

Optionally, the extending direction of the indicator member is consistent with the thread angle of the internal threads.

Optionally, along the axial direction, the one or more indicator members are arranged in a single set or more than two sets, with each set including one indicator member or multiple indicator members circumferentially spaced apart along the bushing.

Optionally, the circumferential extension length of a single indicator member along the bushing ranges from 1/4 to 1/2 of the circumference.

Optionally, the indicator member at least includes:
a first set of indicator members, wherein when the elastic member engages with the first set of indicator members, the interventional device is fully enclosed by the tube connected to the driven member; and
a second set of indicator members, wherein when the elastic member engages with the second set of indicator members, the interventional device is partially released by the tube connected to the driven member.

Optionally, the indicator member includes:
a main body, generally arc-shaped; and
protruding teeth distributed on the inner side of the main body, the protruding teeth being multiple and spaced apart along the extension direction of the main body.

Optionally, the outer periphery of the main body has a smooth structure, or the outer side of the main body is spaced with protruding placeholders.

Optionally, the inner wall of the outer housing directly abuts against the outer periphery of the main body and/or abuts against the placeholders.

Optionally, each set of indicator members is spaced apart from the axial end face of the driving sleeve.

Optionally, the driven member includes:
a base body, at least a portion of which constitutes a sliding part located inside the support body; and
the transmission teeth fixed to the sliding part, wherein the elastic member is connected to either the sliding part or the transmission teeth and extends out of the guide groove, or the elastic member is connected to the transmission teeth via an intermediate member, the intermediate member being located outside the support body.

Optionally, the intermediate member is provided with a positioning hole, through which the intermediate member is connected to the transmission teeth.

Optionally, the intermediate member is tubular and slidably sleeved over the outer periphery of the support body.

Optionally, the elastic member is a bridge arm with two ends fixed to the intermediate member, and the central region of the bridge arm is engageable with the indicator member.

Optionally, the central region of the bridge arm is provided with a protrusion that is engageable with the indicator member.

Optionally, the intermediate member is provided with a deformation release opening arranged between the two ends of the bridge arm.

Optionally, two radial sides of the intermediate member are respectively provided with the elastic member.

Optionally, another set of driving mechanisms is further provided, which includes:
a second handle, located on the proximal side of the first handle and configured to be connected to the proximal end of the inner shaft assembly, the second handle having a first position where it engages with the first handle and a second position slid proximally by a predetermined distance; and
at least one set of locking mechanisms arranged between the first handle and the second handle to maintain their relative positions.

Optionally, a portion of the first handle forms a locking part located at the outer periphery of the driving sleeve, and at least a portion of the locking part forms a radially deformable force-applying portion, and the force-applying portion has a locked state where it interacts with the driving sleeve to prevent its rotation, and a release state where it allows the driving sleeve to rotate.

An operating ring is rotatably sleeved over the periphery of the locking part, and the inner wall of the operating ring is provided with a working portion which is radially inward-protruding and engageable with the force-applying portion, and the working portion moves together with the operating ring to switch the states of the force-applying portion.

The present application further provides a transcatheter delivery system having opposite distal and proximal ends and with axial direction extending therebetween, the interventional device delivery system including a catheter assembly and a control handle connected to the proximal end of the catheter assembly, the control handle including a first handle and multiple sets of driving mechanisms, each set of driving mechanisms being in transmission connection with a corresponding tube of the catheter assembly; wherein one set of driving mechanisms includes:

a second handle, located at the proximal side of the first handle and configured to be connected to the corresponding tube, the second handle having a first position where it engages with the first handle, and a second position where the second handle slides proximally by a predetermined distance from the first position; and
a first locking mechanism and a second locking mechanism disposed between the first handle and the second handle;
wherein the first locking mechanism is configured to maintain the first handle in the first position, and the first locking mechanism includes:
   a first limiting member with an annular structure and radially slidably mounted on the second handle, wherein the inner edge of the annular structure serves as a first locking member that is engageable with the first handle;
   a state component, acting on at least one radial side of the first limiting member, for holding the first limiting member in a locked state engaged with the first handle, or switching it to a release state disengaged the connection; and
   a second locking mechanism configured to hold the second handle in the second position.

Optionally, the second handle includes a second housing, and in the first position, the proximal end of the first handle serves as a connecting section and extends into the distal end of the second housing. In the first locking mechanism, the outer wall of the connecting section is provided with a first locking groove that engages with the first limiting member.

Optionally, the state component includes:
an elastic member, arranged between the second housing and the first limiting member to bias the first limiting member into a locked state; and
a control button, movably inserted in the second housing, wherein the elastic member and the control button act on opposite radial sides of the first limiting member to drive the first limiting member into the release state.

Optionally, the second handle includes a second housing which is connected to an extension sleeve extending into the first handle, and the second locking mechanism includes:
a second limiting member sleeved over the outer circumference of the extension sleeve, and the outer circumference of the extension sleeve is provided with a second locking groove cooperated with the second limiting member, and the second limiting member has a locked state engaged with the extension sleeve and a release state disengaged from the extension sleeve;
a spring, arranged between the first handle and the second limiting member to bias the second limiting member into the locked state; and
a releasing button, fixed to the second limiting member to drive it into the release state.

Optionally, the outer periphery of the extension sleeve is provided with a second locking groove that engages with the second limiting member, and the inner edge of the second limiting member is provided with a second locking member that engages with the second locking groove. Optionally, the state component includes:
a spring, arranged between the first handle and the second limiting member to bias the second limiting member into a locked state; and
a releasing button, fixed to the second limiting member to drive it into the release state.

Optionally, the second handle includes:
a second housing, wherein in the first position, the proximal end of the first handle serves as a connecting section and extends into the distal end of the second housing, and the first locking mechanism acts on the connecting section; and
a tube connector, fixed within the second housing and configured for connecting to the proximal end of the controlled tube.

Optionally, the interior of the extension sleeve defines a guide passageway for the controlled tube.

Optionally, the first limiting member is sleeved over the periphery of the connecting section, and the elastic member and the control button act on opposite radial sides of the first limiting member.

The outer wall of the connecting section is provided with a first locking groove engaged with the first limiting member, and the first locking member engages with the first locking groove.

A guide groove is defined within the second housing, and at least a portion of the first limiting member is slidably fitted in the guide groove.

Optionally, two opposite sides of the connecting section are respectively provided with first locking grooves, and the first locking grooves on opposite sides are axially offset from each other. The first locking mechanism consists of two sets, and the first limiting members of the two sets are stacked side by side along the axial direction. The first locking member of each first limiting member engages with the first locking groove on the corresponding side of the connecting section.

Optionally, the elastic members and control buttons in the two sets are arranged in mirrored configuration; one end of the elastic member in each set abuts against the first limiting member of its own set, while the other end abuts against the control button in the other set.

Optionally, the control button is provided with two positioning structures on its inner side, namely:
a first receiving groove, positioned against the first limiting member of its own set; and
a second receiving groove, positioned against the elastic member in another set.

Optionally, the first limiting member is provided with positioning posts on two opposed sides, namely:
a first positioning post extending into a corresponding first receiving groove;
a second positioning post for the corresponding elastic member to be fitted.

Optionally, a hollow area is defined on the second housing, a portion of the control button is exposed and protrudes outward through the hollow area, and the edge of the control button is provided with an anti-detachment rim within the second housing, and the anti-detachment rim is constrained by the inner edge of the hollow area.

Optionally, the inner edge of the hollow area is ringed with a reinforcing seat extending into the second housing, the reinforcing seat is provided with a stabilizing groove, and at least a portion of the anti-detachment rim extends into the stabilizing groove.

Optionally, the first receiving groove and the second receiving groove are arranged in sequence along the axial direction, and the anti-detachment rim is provided with a lug adjacent to the second receiving groove for engaging with the stabilizing groove.

Optionally, the proximal end of the first handle serves as a connecting section and has a hollow structure for tube extension, the second limiting member is located within the connecting section, and the releasing button extends through and is exposed from the side wall of the connecting section.

In the first position, the second housing covers the releasing button.

In the second position, the releasing button is exposed from the second housing.

Optionally, the spring and the releasing button are located on two opposite sides of the second limiting member.

Optionally, the second limiting member includes a guide post on the side opposite the releasing button, the spring is sleeved over the guide post, and the guide post movably extends through the side wall of the connecting section.

Optionally, the catheter assembly includes an inner shaft assembly and an outer sheath, a radial gap defined between the distal ends of the inner shaft assembly and the outer sheath constitutes a loading zone for accommodating an interventional device, and the inner shaft assembly and the outer sheath are configured to be movable relative to each other so that the loading zone is covered by or exposed from the outer sheath.

The second handle is connected to the inner shaft assembly, and the first handle is connected to the outer sheath.

When the outer sheath is moved distally relative to the first handle, the first handle and the second handle are in the first position, with the distal end of the outer sheath covering the loading zone of the interventional device; and
wherein when the outer sheath is moved proximally relative to the first handle, the first handle and the second handle are in the second position, with the distal end of the outer sheath covering the loading zone of the interventional device.

Optionally, the predetermined distance ranges from 40 mm to 80 mm.

The present application further provides a transcatheter delivery system, which has opposite distal and proximal ends with an axial direction extending therebetween. The interventional device delivery system includes a catheter assembly and a control handle connected to the proximal end of the catheter assembly. The catheter assembly includes an inner shaft assembly and an outer sheath. A radial gap defined between the distal ends of the inner shaft assembly and the outer sheath constitutes a loading zone for accommodating the interventional device. The inner shaft assembly and the outer sheath are configured to be movable relative to each other so that the loading zone is covered by or exposed from the outer sheath.

The control handle includes a first handle and multiple sets of driving mechanisms, each set of driving mechanisms is in transmission connection with a corresponding tube of the catheter assembly; and one set of driving mechanisms includes:
a driving sleeve, rotatably mounted to the first handle;
wherein a portion of the first handle forms a locking part located at the outer periphery of the driving sleeve, and at least a portion of the locking part forms a radially deformable force-applying portion, and the force-applying portion has a locked state where it interacts with the driving sleeve to prevent its rotation and a release state where it allows the driving sleeve to rotate.

An operating ring is rotatably sleeved over the outer periphery of the locking part, and the inner wall of the operating ring is provided with a working portion which is radially inward-protruding and engageable with the force-applying portion, and the working portion moves with the operating ring to switch the states of the force-applying portion.

Optionally, the locking part has a tubular structure, with a portion of its sidewall serving as the force-applying portion, and a hollow area for releasing deformation is defined between the force-applying portion and the surrounding area.

Optionally, there are multiple force-applying portions circumferentially spaced apart along the tubular structure.

Optionally, the circumferential span of the force-applying portion along the tubular structure is at least 1/6 circumference.

Optionally, the inner side of the force-applying portion is provided with first anti-slip teeth along the radial direction of the locking part.

Optionally, a portion of the driving sleeve constitutes an inserting section that engages with the locking part, and the control handle further includes an elastic locking ring sleeved over the inserting section. In the locked state, the force-applying portion abuts against the elastic locking ring and prevents the rotation of the driving sleeve.

Optionally, a mutually cooperating anti-slip structure is provided between the elastic locking ring and the inserting section, and the anti-slip structure includes at least one of the following configurations:
second anti-slip teeth, distributed on the inner wall of the elastic locking ring; and
third anti-slip teeth, distributed on the outer wall of the inserting section.

Optionally, the second anti-slip teeth and the third anti-slip teeth engage with each other.

Optionally, the outer wall of the operating ring is provided with a radially outwardly protruding thumb knob.

Optionally, the force-applying portion is either a single unit or multiple units circumferentially spaced apart along the locking part, and the inner wall of the operating ring is alternately provided with concave zones, and convex zones which serve as the working portion along the circumference.

In the locked state, the outer side of the force-applying portion abuts against the inner convex area.

In the release state, the outer side of the force-applying portion corresponds to the concave area.

Optionally, the concave area and the inner convex area are connected by a slope transition.

Optionally, the first handle includes:
a support body, wherein the movable base is mounted to the support body and slidable along the axial direction, and the driving sleeve is rotatably mounted to the outer periphery of the support body;
an intermediate sleeve, fixed to the support body, wherein the proximal end of the intermediate sleeve is located at the outer periphery of the support body and serves as the locking part; and
a first housing, fixedly enclosing the outer circumference of the intermediate sleeve, with a first gap defined between the first housing and the locking part in a radial direction, wherein the operating ring is rotatably mounted in the first gap and at least partially exposed from the first housing.

The present application discloses a method for releasing a prosthetic heart valve through a delivery system,
the prosthetic heart valve has an expanded configuration and a collapsed state;
the delivery system has opposite distal and proximal ends with an axial direction extending between the distal and proximal ends, wherein the delivery system includes a catheter assembly and a control handle connected to the proximal end of the catheter assembly, the catheter assembly includes an inner shaft assembly and an outer sheath, a radial gap defined between the distal ends of the inner shaft assembly and the outer sheath constitutes a loading zone for accommodating an interventional device, and the inner shaft assembly and the outer sheath are configured to be movable relative to each other so that the loading zone is covered by or exposed from the outer sheath;
the prosthetic heart valve is releasably connected to the loading zone;
the control handle includes a first handle and a second handle slidably fitted together;
the first handle is configured to be connected to the outer sheath;
the second handle is located at the proximal side of the first handle and configured to be connected to the inner shaft assembly, and the second handle has a first position where it engages with the first handle and a second position where the second handle slides proximally by a predetermined distance from the first position;
a first locking mechanism is mounted to the second handle and engageable with the proximal end of the first handle to hold the first handle in the first position;
an extension sleeve, wherein a proximal end of the extension sleeve is connected to the second handle, and a distal end of the extension sleeve extends into the interior of the first handle;
a second locking mechanism is mounted to the first handle and engageable with the extension sleeve to hold the second handle in a second position.

When releasing the prosthetic heart valve, it includes:
maintaining the first handle and the second handle in the first position and delivering the prosthetic heart valve to a designated position;
controlling the outer sheath to slide towards the proximal end relative to the inner shaft assembly via the first handle, exposing the loading zone to release the prosthetic heart valve;
unlocking the first locking mechanism and controlling the inner shaft assembly to slide towards the proximal end relative to the outer sheath via the second handle, so that the outer sheath covers the loading zone, and at this time, the second locking mechanism maintains the first handle and the second handle in the second position; and
driving the catheter assembly to move toward the proximal end away from the designated position via the first handle and the second handle.

Optionally, when controlling the outer sheath to slide towards the proximal end relative to the inner shaft assembly via the first handle, the state of the prosthetic heart valve is confirmed to be as expected upon reaching a predetermined release stage After confirmation, the outer sheath is further driven to fully expose the loading zone.

Optionally, when the prosthetic heart valve reaches the predetermined release stage, the length of the prosthetic heart valve that has been exposed from the outer sheath accounts for 55 % to 80 % of its total axial length.

Optionally, if the state of the prosthetic heart valve fails to meet expectations upon reaching the predetermined release stage, the outer sheath is controlled to slide towards the distal end relative to the inner shaft assembly through the first handle, thereby re-enclosing the prosthetic heart valve, and then the state of the prosthetic heart valve is adjusted as needed.

The transcatheter delivery system provided in the present application provides enhanced operational control in the delivery, release, and retrieval of interventional devices, and improves the operating experience of clinicians and other operators. The specific beneficial technical effects will be further explained in the specific implementations.

### BRIEF DESCRIPTION OF DRAWINGS

FIG. 1 is a first partially exploded view of the present application;
FIG. 2 is a partial cross-sectional view showing the cooperation between the driven member and the driving member in the present application;
FIG. 3 is a schematic structural diagram showing the cooperation between the driven member and the driving member in the present application;
FIGS. 4a-4c are schematic diagrams showing the movement of the outer sheath relative to the inner shaft assembly in the present application;
FIG. 5 is a partial cross-sectional view showing the cooperation between the indicator member and the elastic member in the present application;
FIG. 6 is a schematic structural diagram showing the engagement of the indicator member and the elastic member in FIG. 5;
FIG. 7 is a partial cross-sectional view showing the cooperation between the driving member of the double-layer structure and the supporting body in the present application;
FIG. 8 is a schematic structural diagram showing the driving member of the double-layer structure cooperating with the support body in the present application;
FIG. 9 is a schematic structural diagram showing the elastic member, the indicator member and related components in the present application;
FIG. 10 is a schematic structural diagram showing the engagement of the elastic member and the first set of indicator members in the present application;
FIG. 11 is a schematic structural diagram showing the engagement of the elastic member and the second set of indicator members in the present application;
FIG. 12 is a second partial exploded view of the present application;
FIG. 13 shows a schematic structural diagram of the present application when the outer sheath fully exposes the loading zone;
FIG. 14 shows a schematic structural diagram of the present application when the outer sheath covers the loading zone;
FIG. 15 is a schematic structural diagram showing the first position where the second handle is combined with the first handle in the present application;
FIG. 16 is a schematic structural diagram showing the second position where the second handle has slid towards the proximal end by a predetermined distance in the present application;
FIG. 17 is a partial cross-sectional view of the first locking mechanism in the present application;
FIG. 18 is an exploded view of the first locking mechanism in the present application;
FIG. 19 is a schematic structural diagram showing the second limiting member in a release state according to the present application;
FIG. 20 is a third partial exploded view of the present application;
FIG. 21 is a partial cross-sectional view showing the cooperation between the driven member and the driving member in the present application;
FIG. 22 is a partial cross-sectional view along the direction of the locking part of the present application;
FIG. 23 is a schematic structural diagram showing the locking part in the present application;
FIG. 24 is a cross-sectional view along the direction of the operating ring in the present application;
FIG. 25 is a structural diagram showing the driving sleeve, elastic locking ring, and operating ring in the present application;
FIG. 26 is a structural diagram showing the cooperation between the locking part and the operating ring in the present application;
FIG. 27 is a structural diagram showing the first handle in the present application; and
FIG. 28 is a partial cross-sectional view of the first handle in the present application.

The reference signs in the figures are described as follows:
1. interventional device;
2. catheter assembly; 21. inner shaft assembly; 211. guide head; 212. engaging portion; 22. outer sheath;
3. control handle; 30. first handle; 31. locking part; 311. force-applying portion; 3111. first anti-slip teeth; 312. operating ring; 3121. working portion; 3122. protrusion; 3123. inner convex area; 3124. concave area; 313. tubular structure; 3131. hollow area; 32. support body; 321. guide groove; 33. intermediate sleeve; 34. first housing; 341. indicator window; 342. shielding part; 35. connecting section; 351. first locking groove;
4. driving mechanism; 41. driven member; 41a. movable base; 411. stroke indicator element; 412. transmission teeth; 413. base body; 4131. sliding part; 42. driving member; 42a. driving sleeve; 421. inserting section; 4211. third anti-slip teeth; 4212. limiting flange; 4213. limiting ring; 422. internal threads; 423. bushing; 4231. receiving slot; 4232. limiting step; 424. outer housing;
5. elastic locking ring; 51. second anti-slip teeth;
6. indicator assembly; 60. indicator member; 60a. first set of indicator members; 60b. second set of indicator members; 61. main body; 62. protruding teeth; 63. placeholder;
7. elastic member; 71. protrusion; 72. bridge arm;
8. intermediate member; 81. positioning hole; 82. deformation release opening;
9. second handle; 901. second housing; 9011. guide groove; 9012. hollow area; 9013. reinforcing seat; 9014. stabilizing groove; 902. tube connector;
91, first locking mechanism; 91a, first locking mechanism; 91b, first locking mechanism; 911, first limiting member; 911a, first limiting member; 911b, first limiting member; 9111, first locking member; 9112, first positioning post; 9113, second positioning post; 912, elastic member; 912a, elastic member; 912b, elastic member; 913, control button; 913a, control button; 913b, control button; 9131, first receiving groove; 9132, second receiving groove; 9133, anti-detachment rim; 9134, lug;
92, second locking mechanism; 921, second limiting member; 9211, second locking member; 9212, guide post; 922, spring; 923, releasing button;
93. extension sleeve; 931. second locking groove; 932. anti-detachment structure; 9321. protruding portion; 9322. stopping portion; 933. snap-fit structure; 934. convex rib.

### DESCRIPTION OF EMBODIMENTS

The technical solutions according to the embodiments of the present application will be described clearly and fully in combination with the drawings according to the embodiments of the present application. Obviously, the described embodiments are not all embodiments of the present application, but only part of the embodiments of the present application. Based on the disclosed embodiments, all other embodiments obtained by those skilled in the art without creative work fall into the scope of this application.

It should be noted that, when a component is "connected" with another component, it may be directly connected to another component or may be indirectly connected to another component through a further component. When a component is "provided" on another component, it may be directly provided on another component or may be provided on another component through a further component.

Unless otherwise defined, all technical and scientific terms used herein have the same meaning as commonly understood by a person skilled in the art. The terms in the description of the present application are used to describe specific embodiments, and not to limit the present application. The term "and/or" used herein includes one or more of the listed options in any combinations, or the combination of all of the listed options.

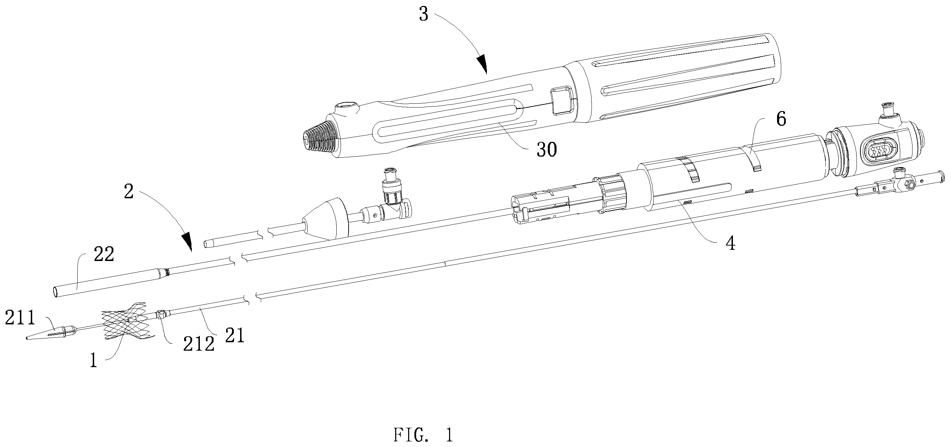

With reference to FIG. 1, the present application discloses a transcatheter delivery system having opposite distal and proximal ends with an axial direction extending between the distal and proximal ends. The distal end may be understood as an end relatively far from an operator, and the proximal end is an end relatively close to the operator.

The transcatheter delivery system (hereinafter also referred to as the delivery system) includes a catheter assembly 2 and a control handle 3 connected to the proximal end of the catheter assembly 2. The control handle 3 is configured to operate the catheter assembly 2 which can load an interventional device 1, with the interventional device 1 being loaded at the distal end of the catheter assembly 2.

The catheter assembly 2 at least includes two tubes that are slidably sleeved over one another, such as the inner shaft assembly 21 and the outer sheath 22 shown in the figures. A radial gap defined between the distal ends of the inner shaft assembly 21 and the outer sheath 22 constitutes a loading zone for accommodating the interventional device 1, and the inner shaft assembly 21 and the outer sheath 22 are configured to be movable relative to each other, so that the loading zone can be covered by or exposed from the outer sheath 22. The distal end of the inner shaft assembly 21 is provided with a guide head 211, which makes the catheter assembly 2 suitable for traversal in the body. When the outer sheath 22 is moved relative to the inner shaft assembly 21, the inner shaft assembly 21 maintains at least a relatively fixed state with respect to the first handle 30, so as to control their relative positions.

The control handle 3 is provided with an indicator assembly 6, which may be connected to the control handle 3 as a separate component or integrated as a single piece. When the outer sheath 22 is moved to a predetermined relative position relative to the inner sheath component 21, the indicator assembly 6 responds to the relative position and provides direct feedback to the operator.

In practice, during the movement of the outer sheath 22 relative to the inner shaft assembly 21, it may be necessary to provide prompts for multiple relative positions. That is, there may be a first relative position and a second relative position sequentially arranged along the axial direction, and the indicator assembly can respond to each of the two relative positions and provide direct feedback to the operator.

Since the operator will continuously hold the control handle during the procedure, the indicator assembly can provide a tactile feedback in the form of vibration and/or a sound feedback, allowing the operator to promptly receive the haptic response signal. This means that feedback of haptic sensation, sound, or a combination of both is provided for the predetermined relative position, which ultimately achieves the purpose of providing an intuitive haptic response signal to the operator. When the outer sheath tube 22 moves to the predetermined relative position, thereby eliminating the repeated redundant observation brought by the visual signal.

In order to achieve the purpose of responding to multiple relative positions, the indicator assembly includes first and second components interacting with each other at each relative position, wherein multiple first components are arranged at multiple predetermined relative positions correspondingly and spaced apart along the axial direction. For each relative position, the second component interacts with the first component at a various axial position, that is, as the second component slides axially, each encounter with the first component produces one response, thereby achieving multiple responses.

The second component and the first component, in the state of interaction, engage with each other in the radial direction of the control handle to allow their axial movements, allowing for more flexible configuration of components and rational use of space.

Preferably, the first component is designed as an indicator member, while the second component is an elastic member. The elastic member and the indicator member engage with each other (i.e., engage with each other radially along the control handle) when aligned. The deformation of the elastic member at least allows the indicator member to override the elastic member, thereby producing a sound effect each time the indicator member overrides the elastic member. This ultimately conveys a tactile and/or sound change effect to the operator.

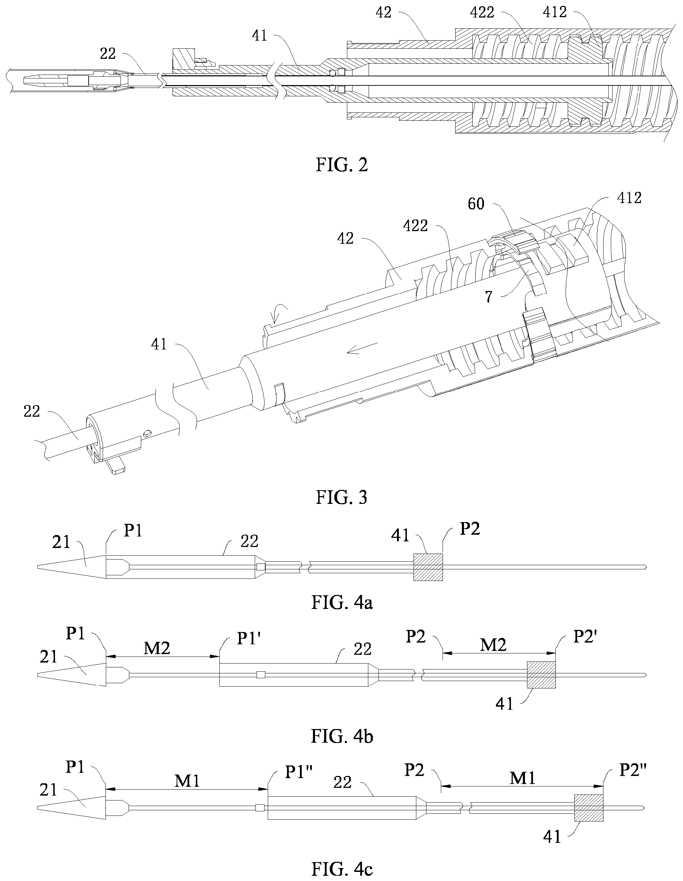

In some embodiments, referring to FIGS. 2 to 3, the control handle 3 includes a first handle 30 and multiple sets of driving mechanisms installed on the first handle 30. Each set of driving mechanisms is in transmission connection with the corresponding tube in the catheter assembly 2 to achieve the operation of the set of driving mechanisms on the specific tube, and finally completes the manipulation such as delivery, release, and retrieval of the interventional device.

One set of driving mechanisms 4 includes a driven member 41 and a driving member 42 sleeved over the periphery of the driven member 41. The driven member 41 is mounted to the first handle 30 and slidable along the axial direction, and the driven member 41 is configured to be connected to the corresponding tube (i.e., the outer sheath 22). The driving member 42 is rotatably mounted to the first handle 30. The driving member 42 and the driven member 41 are in threaded transmission connection, thereby converting the rotation of the driving member 42 into the axial motion of the driven member 41 (i.e., the outer sheath 22). The driving member 42 is provided with internal threads 422, and the driven member 41 is provided with transmission teeth 412. The internal threads 422 are in threaded transmission connection with the transmission teeth 412 on the driven member 41, so that the operator can drive the driven member 41 and the connected tube to slide by rotating the driving member 42.

The control handle 3 further includes one or more indicator members 60 connected to the driving member 42 and an elastic member 7 connected to the driven member 41, wherein the elastic member 7 may be connected to the driven member 41 as a detachable structure by means of, for example snap-fitting, plugging-in, or nesting connection. The structure and the connection means are also applicable to the indicator member 60.

When the elastic member 7 moves axially together with the driven member 41 to a position corresponding to the indicator member 60, where the position of the indicator member 60 can be understood as a predetermined interval position, the elastic member 7 and the indicator member 60 interact with each other to provide a tactile feedback and/or a sound feedback, that is, a response corresponding to the predetermined interval position. This ultimately achieves the effect of providing the tactile feedback and/or sound feedback when the outer sheath moves relatively to the predetermined interval position.

The elastic member 7 and the indicator member 60, in the state of interaction, engage with each other in the radial direction of the control handle to release their axial movement, allowing for more flexible configuration of components and rational use of space.

When the outer sheath 22 moves to a predetermined relative position relative to the inner shaft assembly 21, the elastic member 7 and the indicator member 60 engage with each other to provide the tactile feedback and/or sound feedback.

In practice, during the movement of the outer sheath 22 relative to the inner shaft assembly 21, it may be necessary to provide prompts for multiple relative positions, including at least a first relative position and a second relative position sequentially along the axial direction. The elastic member 7 and the indicator member 60 can respond to each of the relative positions and provide direct feedback to the operator.

Referring to FIG. 4a, in the first relative position, the position of the outer sheath 22 is denoted as P1, and the position of the driven member 41 connected to the outer sheath 22 is denoted as P2. Referring to FIG. 4c, when the outer sheath fully releases the loading zone, i.e., the outer sheath 22 fully exposes the engaging portion 212 of the inner shaft assembly 21, the position of the outer sheath 22 is denoted as P1", and the position of the driven member 41 is denoted as P2". Referring to FIG. 4b, the second relative position is an important position (which may be a partially released position, etc.) between the first relative position and the fully released position, at this time, the position of the outer sheath 22 is denoted as P1', and the position of the driven member 41 is denoted as P2'.

The total stroke of the outer sheath 22 relative to the inner shaft assembly 21 is M1 (i.e., the distance between P1 and P1"). Since the outer sheath 22 is connected to the driven member 41, the total stroke of the driven member 41 is M1, too. The stroke of the outer sheath between the first relative position and the second relative position is M2 (i.e., the distance between P1 and P1'), and M2 is at least 1/3 of M1.

At a predetermined relative position relative to the inner shaft assembly 21, the relative position is not limited to a single time point, but may correspond to a short stroke of the outer sheath 22 (along the axial direction). It may correspond to multiple rotation cycles of the driving sleeve, so as to allow the indicator member to provide multiple responses during this stroke. Preferably, the indicator member provides multiple responses when the rotation amplitude of the driving sleeve is less than or equal to one circle.

The driving member 42a may adopt a driving sleeve with a hollow tubular structure, which facilitates the operator to apply a circumferential rotational force. The internal threads may be set on the inner wall of the driving sleeve. The driven member may be a movable base 41a, and at least one transmission tooth is arranged on the movable base to ensure engagement with the internal threads to achieve the purpose of transmission.

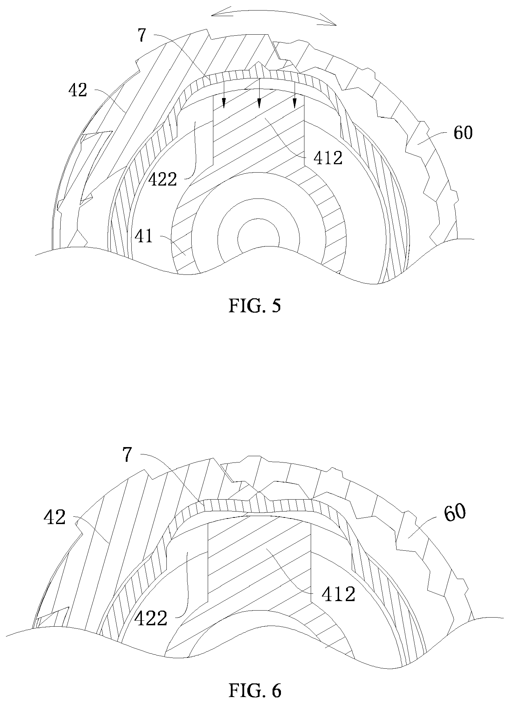

Referring to FIGS. 5 to 6, the indicator member 60 is located on the inner side of the driving member 42, and the indicator member 60 is spaced apart from the movement path of the transmission teeth 412. It can be understood that the movement paths of the indicator member 60 and the transmission teeth 412 in the radial space do not interfere with each other, so the indicator member 60 will not affect the threaded transmission connection between the driving member 42 and the driven member 41.

The elastic member 7 and the indicator member 60 engage with each other when aligned, and the deformation of the elastic member 7 at least allows the indicator member 60 to rotate with the driving member 42 and override the elastic member 7. As shown in the figure, the circumferential highest point of the elastic member 7 is higher than the circumferential lowest point of the indicator member 60. The two components engage with each other circumferentially when in positional engagement. The deformation of the elastic member 7 at least allows the indicator member 60 to rotate with the driving member 42 and override the elastic member 7, so that the indicator member 60 overrides the elastic member 7 to produce a sound effect, ultimately conveying the operator with tactile and/or sound change effect.

In order to provide the operator with more timely and rapid feedback on the critical position or progress of the interventional device operation, this embodiment adopts cooperating structures of the indicator member and the elastic member, supplemented by a tactile or sound feedback function, to directly convey the operation progress to the operator, thereby enabling the operator to judge the operation progress more accurately and sensitively.

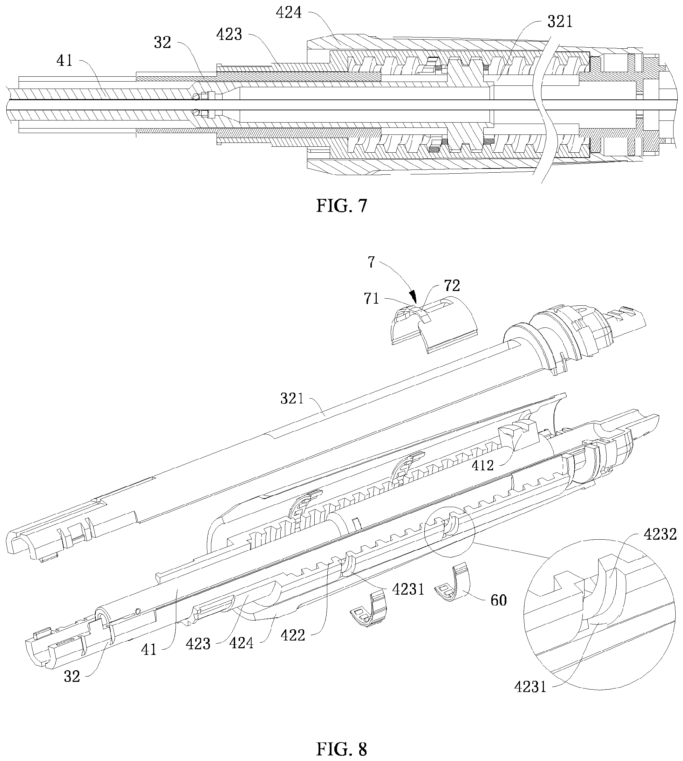

Referring to FIGS. 7 to 8, to further enhance the stability of the threaded transmission connection between the driving member 42 and the driven member 41, the first handle 30 at least includes a support body 32, which is provided with a guide groove 321 extending in the axial direction. The driven member 41 is slidably fitted in the guide groove 321, the driving member 42 is rotatably sleeved over the outer periphery of the support body 32, and the guide groove 321 serves to prevent the circumferential rotation of the driven member 41, that is, the driven member 41 can only slide in the axial direction by the prevention effect of the guide groove 321.

The support body 32 is generally tubular (with an interior that other tubes can pass through), and the guide groove 321 penetrates radially through the support body 32. The driven member 41 features transmission teeth 412 on two radial sides of the support body 32, which extend out of the guide groove 321 and threadedly engage with the driving member 42. This ensures that the transmission teeth 412 and the internal threads 422 are stably engaged for transmission, reducing unnecessary deflection or misalignment.

With respect to the structural design of the driving member, it may adopt a single-layer structure or a double-layer structure. The single-layer structure has internal threads engageable with the transmission teeth 412 and a space that secures the indicator member 60, making it suitable for use in the aforementioned embodiment.

To facilitate machining and assembly, as shown in FIG. 7, the driving member 42 may alternatively adopt a double-layer structure with a bushing 423 and an outer housing 424, and a cooperating anti-rotation structure is arranged between the outer housing 424 and the bushing 423, so that the outer housing 424 is fixedly sleeved over the outer periphery of the bushing 423, and the anti-rotation structure includes at least one of the following:
a limiting groove located at either the inner wall of the housing 424 or the outer wall of the bushing 423; and
a limiting rib located on either the other of the inner wall of the housing 424 or the outer wall of the bushing 423.

The side wall of the bushing 423 is provided with receiving slots 4231, and the indicator member 60 is located in the corresponding receiving slot 4231. The inner wall of the bushing 423 is provided with internal threads 422 for transmission connection with the driven member 41.

Furthermore, since the side wall of the bushing 423 is provided with the receiving slots 4231 for accommodating the indicator member 60, to prevent the operator from accidentally touching the indicator member directly, the housing 424 in the double layer structure of the active member covers the receiving slots 4231.

Given the relatively complex structures of the indicator member and the bushing, if the two components are formed in one piece, the manufacturing process will be too complicated and increases the production cost. Therefore, in this application, the indicator member 60 and the bushing 423 are formed in separate fixed structures, meaning that the indicator member 60 is fixed to the bushing 423 in a manner known in the art.

Preferably, a limiting step 4232 is provided on the inner edge of the receiving slot 4231, and the edge of the indicator member 60 is fixedly seated on the limiting step 4232. On this basis, adhesion or other fixation means may be used as needed. The limiting step 4232 may be a portion of the thread line of the internal threads 422, thereby eliminating the need for additional processing steps.

The cooperation between the indicator member 60 and the elastic member 7 is not limited to a single time point, and preferably corresponds to a short stroke of the driven member, allowing multiple prompts during this process to ensure operator awareness. Therefore, the indicator member 60 itself has a corresponding extension length in both the axial and circumferential directions of the support body. To accommodate this, the receiving slot 4231 is configured as a slot-shaped hole, with its shape corresponding to that of the indicator member 60. The extension direction of the slot-shaped hole is consistent with the thread angle of the internal threads, which precisely adapts to the axial movement component of the driven member, so as to ensure multiple, stable prompts during the cooperation between the indicator member 60 and the elastic member 7.

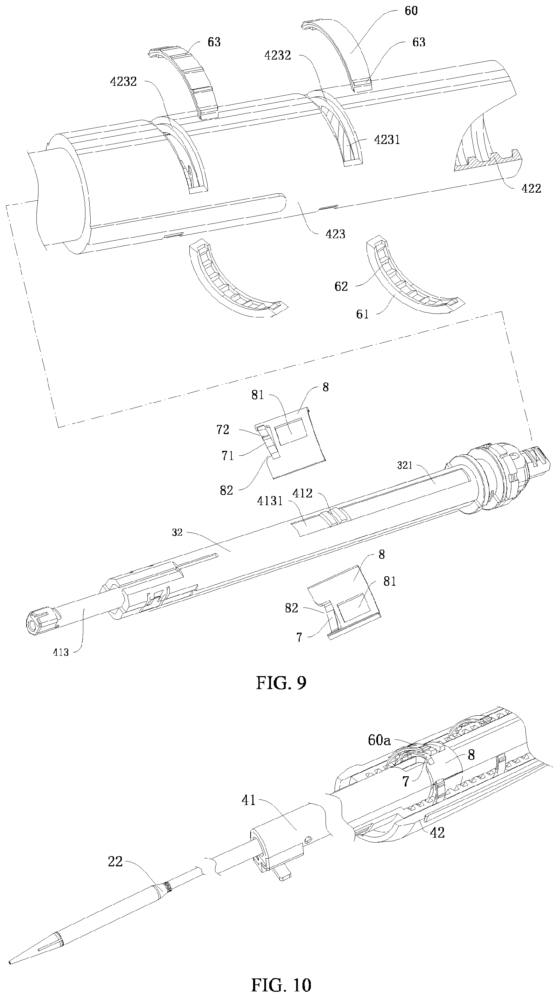

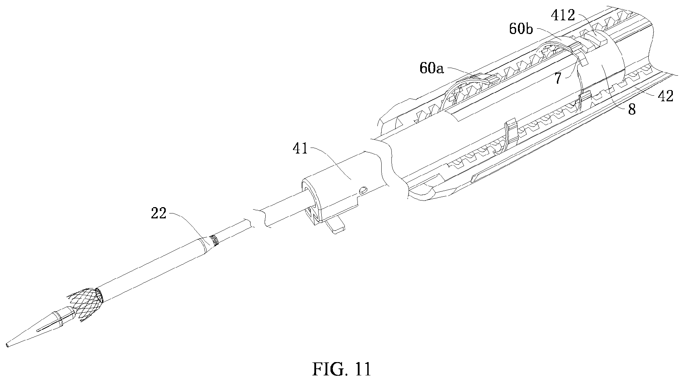

Referring to FIGS. 9 to 11, the elastic member is an important component that is engageable with the indicator member to produce a sound prompting effect. In order to fix the elastic member better, the driven member 41 of the present application includes a base body 413, and at least a part of the base body 413 is a sliding part 4131 located inside the support body 32. The transmission teeth 412 are fixed to the sliding part 4131, and the elastic member 7 can be directly or indirectly connected to the sliding part 4131.

The following provides an improved method for the indirect connection between the elastic member 7 and the sliding part 4131. As shown in FIG. 9, the elastic member 7 is fixed to the sliding part 4131 via an intermediate member 8, and the intermediate member 8 is located outside the support body 32. Therefore, the installation of the intermediate member 8 is not limited to the narrow space between the sliding part 4131 and the support body 32, but utilizes the larger space outside of the support body 32, thereby further enhancing the structural stability.

The intermediate member 8 is provided with a positioning hole 81, and is fixedly sleeved over the transmission teeth 412 through the positioning hole 81, so that the intermediate member 8 moves synchronously with the transmission teeth 412. That is to say, the connection between the elastic member 7 and the sliding part 4131 is achieved.

The intermediate member 8 is tubular and is slidably sleeved over the outer periphery of the support body 32, so that the inner wall of the intermediate member 8 fits better with the support body 32.

To further enhance the elasticity and structural strength of the elastic member itself, the elastic member 7 is a bridge arm 72 with two ends fixed to the intermediate member 8. Since the bridge arm 72 has a large elastic deformation, the central region of the bridge arm 72 is in cooperation with the indicator member, and the indicator member can smoothly override the elastic member 7.

Since the setting direction of the arc-shaped indicator member is consistent with the thread angle of the internal threads, the extension angle of the bridge arm 72 in the lengthwise direction is also consistent with the thread angle of the internal threads, to ensure smoother cooperation between the bridge arm and the indicator member.

The central region of the bridge arm 72 is provided with a protrusion 71 that is engageable with the indicator member. More specifically, protruding teeth 62 provided on the indicator member cooperate with the protrusion 71, thereby minimizing the deformation of the elastic member 7 when the indicator member overrides the elastic member 7.

The intermediate member 8 is provided with a deformation release opening 82 located between the two ends of the bridge arm 72. When the bridge arm 72 is squeezed by the indicator member, most of the acting force is releasable through the deformation release opening 82, preventing the bridge arm from being damaged by excessive deformation.

The intermediate member 8 is equipped with elastic members 7 on two radial sides, so that the elastic members 7 on two sides can engage with the corresponding indicator member and produce a sound prompting effect, which further enhances the overall prompting effect of the present application.

With respect to the details of the cooperation between the protruding teeth 62, the protrusion 71, and the bridge arm, it is suggested that the radial height of the tooth tip of the protruding teeth 62 in the indicator member is not lower than the root of the thread groove of the internal threads 422, so that the movement paths of the protruding teeth 62 and the internal threads 422 do not interfere with each other. The radial height of the protrusion 71 is intermediate between the tooth tip and the tooth root of the protruding teeth 62. Thus, when the protrusion 71 reaches the position corresponding to the tooth root of the protruding teeth 62, the bridge arm 72 can restore its initial shape.

The bridge arm 72 is disposed within the thread groove of the internal threads 422, and has a deformed energy-storing state and a reset state. When the bridge arm 72 and the protrusion 71 abut against the root of the thread groove, the bridge arm 72 is deformed under force and stores elastic energy, which means that the bridge arm 72 at this time is in the deformed energy-storing state. When the position of the protrusion 71 corresponds to the root of the protruding teeth 62, the bridge arm 72 returns to its original shape due to its own elastic restoring force, that is, the bridge arm 72 at this time is in the reset state.

With the continuous rotation of the driving member 42, the protrusion 71 moves further in the radial direction and continues to contact the indicator member. The protrusion 71 repeatedly reaches positions corresponding to the tip or root of the protruding teeth 62, so that the protrusion 71 switches back and forth between the deformed energy-storing state and the reset state. Each time the switch occurs, the protruding teeth 62 override the protrusion 71, producing a sound effect.

With respect to the number and placement requirements of the indicator member, the present application specifies that one or more sets of indicator members are arranged along the axial direction of the control handle. The indicator members of each set may consist of one or multiple indicator members circumferentially spaced apart along the bushing 423, as shown in FIG. 9, two sets of indicator members are circumferentially spaced apart along the bushing 423, so that the indicator members produce at least one set of sound effects, conveying the change effect of tactile and sound to the operator.

The circumferential extension length of a single indicator member along the bushing ranges from 1/4 to 1/2 of the circumference, ensuring that the sound effect produced by each set of the indicator member can last long enough for the operator to recognize and respond.

To further enhance the strength and sound prompt effect of the indicator member, as shown in the figure, the indicator member includes a main body 61 that is curved as a whole and protruding teeth 62 distributed on the inner side of the main body. There are multiple protruding teeth 62 spaced apart along the extension direction of the main body 61. The multiple protruding teeth not only enhance the structural strength of the main body, but also, when engaged with the elastic member, the multiple protruding teeth produce multiple rhythmic sound prompts. This draws more attention to the operator and achieves a better sound prompt effect.

To make the outer periphery of the indicator member fit better with the outer housing, the outer periphery of the main body 61 features a smooth structure. Alternatively, if there is a large gap defined between the outer housing and the outer periphery of the main body, protruding placeholders 63 are spaced apart along the outer side of the main body 61, thereby enhancing more stable placement position of the indicator member.

The inner wall of the outer housing 424 directly abuts against the outer periphery of the main body 61 and/or the placeholder 63, so that the inner wall of the outer housing 424 can be directly fit against the main body 61 of the indicator member, or to abut via the placeholder 63. The shape of the inner wall of the housing 424 is not limited by the fixing requirements of the indicator member, and the shape of the inner cavity is not limited to a straight cylindrical shape.

Each set of indicator members 60 is spaced apart from the axial end face of the driving member 42, that is, along the axial direction, each set of indicator member is arranged between the proximal and distal ends of the driving member 42, rather than at these two ends.

Given the stock of the outer sheath between the first relative position and the second relative position being M2, the distance between two sets of indicator members corresponding to the first relative position and the second relative position in the axial direction of the bushing 423 is also M2.

In addition, the operator needs to be prompt and timely when the interventional device is about to reach a certain special state. Therefore, the change in the relative position of the indicator member to the driven member actually corresponds to the release process of the interventional device.

The outer sheath 22, during the relative movement with respect to the inner shaft assembly 21, at least includes:
a released position, referring to that the outer sheath 22 moves to the proximal end relative to the inner shaft assembly 21 to fully expose the loading zone, at which point the interventional device is allowed to detach from the delivery system to be fully released;
a full retrieval position or initial position, referring to that the loading zone and the interventional device are completely covered by the outer sheath 22, or the interventional device can be further retracted from the body after being released, at which time the distal end of the outer sheath is interconnected with the guide head 211; and
a partially released position, referring to that the outer sheath 22 partially exposes the loading zone, the distal end of the interventional device has been released, but the proximal end, especially the engaging portion between the mounting head, is still enclosed and bound by the outer sheath 22. Generally, in case the interventional device is a valve system, all the leaflets of the valve have been exposed by the outer sheath, and the leaflets can function normally in a partially release state. Typically, 60% to 70% of the total length of the valve has been exposed by the outer sheath. At this time, the outer sheath 22 is still movable to the distal end to retrieve the interventional device 1. If the release progress exceeds this position, it will be difficult or even impossible to retrieve the interventional device 1.

Among them, the interventional device 1 is generally engaged with the engaging portion of the inner shaft assembly 21 through the connecting ear. In the aforementioned partially released position, the connecting ear and the engaging portion are always enclosed by the distal end of the outer sheath 22.

Additionally, in the partially released position, the proximal portion of the interventional device can be restrained at this time, or the distal portion of the interventional device can be restrained (i.e., the proximal portion is released first) according to the release order of the interventional device.

Based on the above practical needs, as shown in FIGS. 10 to 11, in the present application, two sets of indicator members are arranged corresponding to the full retrieval position and the partially released position of the interventional device. When the elastic member 7 engages with the first set of indicator member 60a, the outer sheath 22 connected to the driven member 41 completely receives the interventional device, and at this time the operator is prompted not to drive the outer sheath to move to the distal end any more.

The second set of indicator member 60b is located on the proximal side of the first set of indicator member 60a. When the elastic member abuts against the second set of indicator member 60b, the outer sheath connected to the driven member partially releases the interventional device, that is, at this time, the proximal end of the interventional device has not yet been completely released from the conveying system. For example, in the partially released position, the connecting ear of the interventional device and the engaging portion 212 of the inner shaft assembly 21 are still enclosed by the distal end of the outer sheath. At this time, if it is necessary to retrieve the interventional device, the outer sheath can be pushed towards the distal end.

However, once the outer sheath moves towards the proximal end and overrides the partially released position, the interventional device is completely exposed and released, and cannot be retrieved solely by pushing the outer sheath. Therefore, it is necessary to provide prompts to the operator for the partially released position when releasing the interventional device.

Whether performing retrieval, or after the proper release of the interventional device, it is necessary to push the outer sheath towards the distal end. When the elastic member 7 abuts against the first set of indicator members, the outer sheath 22 connected to the driven member completely receives the interventional device. At this time, the operator is prompted not to drive the outer sheath towards the distal end to avoid excessive compression of the guide head and deformation of the distal end, which may pose a safety hazard.

After properly releasing the interventional device, the control handle needs to push the outer sheath towards the distal end to restore the catheter assembly to its initial position, i.e., a state suitable for traversal in the body. Some control handles employ a single threaded drive structure, that is, relative to the release process, the movement of the outer sheath towards the distal end is driven by the reverse rotation of the driving sleeve, but the efficiency is low.

In order to address the aforementioned issues and improve the adjustment efficiency of the catheter assembly, in some embodiments described below, the control handle may adopt a direct drive method to quickly retract the inner shaft assembly towards the proximal end, thereby rapidly restoring the catheter assembly to its initial position.

Some of the following embodiments further provide improvements of the locking mechanism combining with the rapid retraction function of the tube, such as the aforementioned catheter assembly and the first handle, which can be combined with the above embodiments.

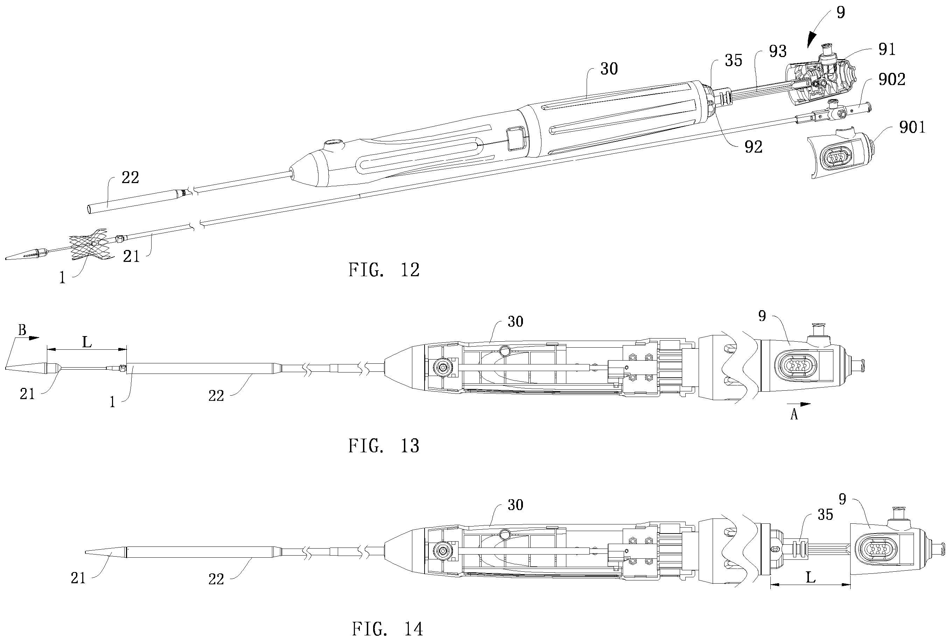

In some embodiments, referring to FIGS. 12 to 14, the control handle 3 includes a first handle 30 connected to the outer sheath 22 and multiple sets of driving mechanisms, each set of driving mechanisms being in transmission connection with a corresponding tube fitting in the catheter assembly 2.

One set of driving mechanisms includes a second handle 9 and at least a locking mechanism disposed between the first handle 30 and the second handle 9. The second handle 9 is located at the proximal side of the first handle 30 and configured to be connected to the inner shaft assembly 21. The first handle 30 is connected to the outer sheath 22 and drives the outer sheath 22 to slide axially relative to the inner shaft assembly 21.

When the outer sheath 22 moves relative to the inner shaft assembly 21, the inner shaft assembly 21 is fixedly connected to the outer sheath 22, and the second hand 9 is fixedly connected to the first handle 30, so as to control the relative position of each tube.

The second handle 9 has switchable first and second positions, the first position corresponds to a position where the second handle 9 is combined with the first handle 30, and the second position corresponds to a position where the second handle 9 is detached from the first handle 30 and can slid proximally by a predetermined distance from the first position, that is, the inner shaft assembly 21 slides towards the proximal end axially by a predetermined distance. The locking mechanism is configured for switching and holding between the first position and the second position, i.e. switching the axial relative position of the second handle 9 to the first handle 30 and keeping them relatively stable.

A locking mechanism is arranged between the first handle and the second handle, specifically including a state component and a limiting member. The limiting member has an annular structure, which is sleeved and radially slidably installed on one of the first and second handles 30, 9. The inner edge of the annular structure serves as a locking member that is engageable with the other one of the two handles. For example, the inner edge of the annular structure may have protruding ribs that serve as the locking member. The locking member abuts against the corresponding handle, or extends into a hole, slot or other structure on the corresponding handle, so as to lock the corresponding handle. The state component can apply its own restoring force to the radially opposite sides of the limiting member, for holding the limiting member in the locked state as well as switching it to the release state.

The locking mechanism includes a first locking mechanism 91 for holding the first handle 30 in the aforementioned first position and a second locking mechanism 92 for holding first handle 30 in the second position. The first locking mechanism 91 is engageable with the proximal end of the first handle 30 to maintain the second handle 9 in the first position. At this time, the second handle 9 is axially relatively fixed to the first handle 30 by the first locking mechanism 91.

In the first locking mechanism 91, the limiting member is a first limiting member 911 movably mounted in the second housing 901 and, in the first position, is located at the outer periphery of the connecting section 35. The state component includes an elastic member 912 and a control button 913. The elastic member 912 acts between the second housing 901 and the first limiting member 911 to bias the first limiting member 911 into a locked state. The control button 913 is movably inserted in the second housing 901 and works in conjunction with the first limiting member 911 to drive the first limiting member 911 into a release state.

In the second locking mechanism 92, the limiting member is a second limiting member 921 sleeved over the outer periphery of the extension sleeve, and the state component includes a spring 922 and a releasing button 923. The spring 922 acts between the first handle 30 and the second limiting member 921 to bias the second limiting member 921 into a locked state, and the releasing button 923 is fixed to the second limiting member 921 to drive the second limiting member 921 into a release state. Some of the following embodiments provide improvements to the first locking mechanism and the second locking mechanism.

In some embodiments, one set of driving mechanisms includes a second handle 9, an extension sleeve 93, a first locking mechanism 91, and a second locking mechanism 92, wherein the second handle 9 is located at the proximal side of the first handle 30 and configured to be connected to the inner shaft assembly 21. The relative movement between the second handle 9 and the first handle 30 is also synchronously reflected as the relative movement of the inner shaft assembly 21 and the outer sheath 22. The proximal end of the extension sleeve 93 is fixedly connected to the second handle 9 by means of such as snap connection, and the proximal end of the extension sleeve 93 is engageable with the first locking mechanism 91. The distal end of the extension sleeve 93 extends into the interior of the first handle 30 and is engageable with the second locking mechanism 92.

The second handle 9 has switchable first and second positions. The first position corresponds to a position where the second handle 9 is combined with the first handle 30, and the second position corresponds to a position where the second handle 9 is detached from the first handle 30 and can be slid proximally by a predetermined distance. The predetermined distance can be constrained by the cooperation between the extension sleeve 93 and the second locking mechanism 92, and the second locking mechanism 92 is configured to maintain the second handle 9 in the second position. Among them, the predetermined distance is in the range of 40mm to 80mm to accommodate catheter assemblies of different specifications and expand the disclosure scope.

The first locking mechanism 91 is mounted to the second handle 9 and engageable with the proximal end of the first handle 30 to hold the second handle 9 in the first position, that is, the second handle 9 is axially fixed relative to the first handle 30 by the first locking mechanism 91.

When the locking effect of the first locking mechanism 91 is released, the second handle 9 is switchable to the second position, that is, the second handle 9 slides towards the proximal end relative to the first handle 30, and the extension sleeve 93 also slides the predetermined distance together with the second handle 9 toward the proximal end.

When the second handle 9 slides to a predetermined position, the second locking mechanism 92 acts on the extension sleeve 93 and locks it, thereby maintaining the second handle 9 in the second position. This allows the second handle 9 to drive the inner shaft assembly 21 to retract quickly axially towards the proximal end and maintain a relative position.

When the catheter assembly 2 traverses in the body, the distal end of the outer sheath 22 abuts against or slightly encloses the proximal end of the guide head 211 (i.e., in the initial position described above) to prevent the interventional device from being exposed. At this time, the second handle 9 is in the first position.

After the interventional device 1 is completely released, the loading zone is fully exposed (i.e., in the released position described above), the distance between the distal end of the outer sheath 22 and the guide head is L, and the catheter assembly 2 needs to be retracted from the body at this time. If the outer sheath 22 is driven to move to the distal end and reaches the guide head by a threaded mechanism, the efficiency would be low. At this time, the first locking mechanism 91 can be unlocked, and the second handle 9 can be driven to quickly retract the inner shaft assembly 21, so that the distal end of the outer sheath 22 abuts against or slightly encloses the proximal end of the guide head (as shown in FIG. 14). At this time, the second handle 9 is in and remains in the second position. During this process, the stroke of the second handle 9 is also L. Finally, the first handle 30 and the second handle 9 are synchronously moved to retract all the tubes from the body. During the retraction, the second locking mechanism 92 is used to prevent the relative displacement of the first handle and the second handle, so as to avoid penetration obstruction caused by exposure of the proximal end of the guide head.

Compared with the single threaded drive method of the catheter assembly in the prior art, the present application introduces a novel drive method based on the existing single drive, which assists in the stage of retracting the inner shaft assembly 21 towards the proximal end, enabling rapid retraction of the inner shaft assembly 21 towards the proximal end. This allows the outer sheath 22 and the inner shaft assembly 21 to quickly restore to a state suitable for traversal in the body.

In the present application, the relative positions of the outer sheath 22 and the inner shaft assembly 21 are directly adjusted by altering the relative positions of the first handle and the second handle. When the operator quickly switches the second handle to the second position, the inner shaft assembly 21 is synchronously quickly retracted, which also improves the adjustment efficiency of the relative positions between the tubes, and achieves a more manipulative effect and operational experience.

The two sets of locking mechanisms are used to hold the first handle and the second handle in different relative positions, thereby locking the corresponding positions of the outer sheath 22 and the inner shaft assembly 21, so as to avoid the adverse effects caused by operating errors. In addition, the distance of the second handle to be quickly retracted can be constrained by the extension sleeve, and the relative position can be maintained by the second locking mechanism 92. Subsequently, all the tubes can be synchronously driven to retract from the body through the entire control handle, achieving rapid and timely adjustment of the catheter assembly.

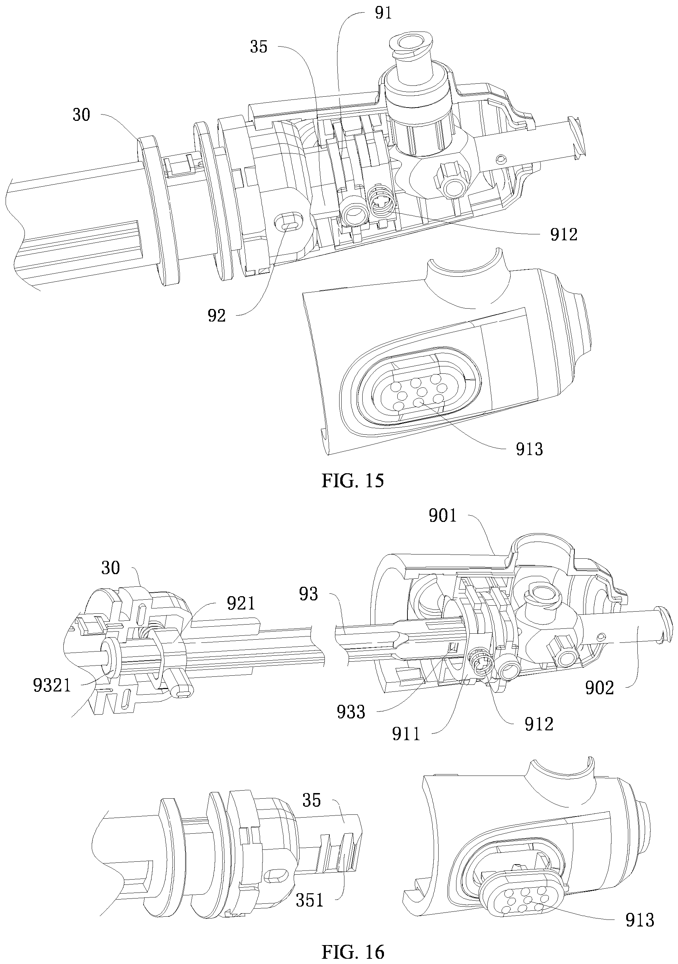

Referring to FIGS. 15 and 16, the second handle 9 includes a second housing 901 and a tube connector 902 fixed within the second housing 901. The tube connector 902 is configured to dock with the proximal end of a controlled tube (i.e., the inner shaft assembly 21), and the proximal end of the extension sleeve 93 can be fixedly connected to the tube connector 902 through a snap-fit structure 933, and the interior of the extension sleeve 93 defines a guide passageway for the inner shaft assembly 21.

In the first position, the proximal end of the first handle 30 serves as the connecting section 35 and extends into the distal end of the second housing 901. The first locking mechanism 91 acts on the connecting section 35 to establish a locked state where the two components engage with each other, and a release state where the two components disengage from each other.

The first locking mechanism 91 includes an elastic member 912, a first limiting member 911 movably mounted in the second housing 901, and a control button 913 movably inserted in the second housing 901.

The first limiting member 911 has a locked state engaged with the connecting section 35 and a release state disengaged from the connecting section 35. The locked state may be understood as the first limiting member 911 being engaged with the connecting section 35 to prevent the second housing 901 from axially disengaging from the first handle 30. The release state may be understood as the first limiting member 911 is disengaged from the connecting section 35, thereby allowing the operator to move the second handle 9 further proximally relative to the first handle 30 for directly driving the controlled tube. Since no transmission components such as threads or gears are required, the controlled tube can be quickly retracted toward the proximal end while pulling the second handle 9.

The outer wall of the connecting section 35 is provided with a first locking groove 351 that is engageable with the first limiting member 911. In the locked state, the first limiting member 911 is engaged with the first locking groove 351 for axial positioning.

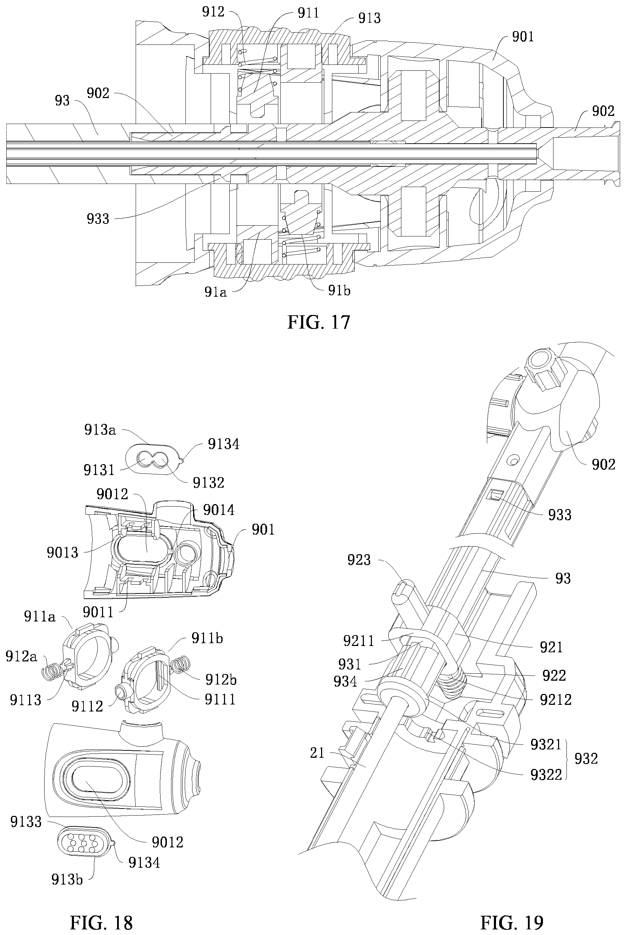

Referring to FIGS. 17 to 18, the elastic member 912 acts on the first limiting member 911 to bias the first limiting member 911 to remain in a locked state, such that in case no external force is applied, the initial state of the first limiting member 911 is the locked state (which can improve safety), and when the operator needs to switch the first limiting member 911 to the release state, he only needs to overcome the elastic force exerted by the elastic member 912.

The control button 913 and the first limiting member 911 are in conjunction with driving the first limiting member 911 into the release state. The operator can switch the state of the first limiting member 911 by directly applying force to the control button 913.

Preferably, the first limiting member 911 has an annular structure sleeved over the periphery of the connecting section 35. The elastic member 912 and the control button 913 act on two opposite radial sides of the first limiting member 911. When the operator presses the control button 913, the pressing force is directly transmitted to the first limiting member 911, causing displacement and entering the releasing state The annular structure not only fully utilizes the space inside the second housing, but also provides the first limiting member with sufficient structural strength.

The inner edge of the annular structure of the first limiting member 911 has a first locking member 9111 that engages with the first locking groove 351. The engagement of the first locking member 9111 and the first locking groove 351 makes the locked state more stable.

The second housing 901 is provided with a guide groove 9011, and at least a portion of the first limiting member 911 is slidably fitted into the guide groove 9011. This further ensures the stability of the movement path of the first limiting member 911 and the smoothness of state switching.

The connecting section 35 has a first locking groove 351 on each of its two opposite sides, and the first locking grooves 351 on the two opposite sides are axially offset from each other. The first locking mechanism (first locking mechanism 91a, first locking mechanism 91b) consists of two sets, and the first limiting members (first limiting member 911a, first limiting member 911b) in the two sets are stacked side by side along the axial direction. The first locking member 9111 of each first limiting member engages with the first locking groove 351 on the corresponding side of the connecting section 35. The two sets of first limiting members, which are stacked in parallel, lock the connecting section 35 from opposite sides, resulting in a more even distribution of force and ensuring a reliable locking effect.

The two sets of elastic members (elastic member 912a, elastic member 912b) and control buttons (control button 913a, control button 913b) are arranged in mirrored configuration, each corresponding to respective first limiting members (first limiting member 911a, first limiting member 911b).

One end of the elastic member in each set abuts against the first limiting member in the set, and the other end abuts against the control button in the other set. As shown in the figure, one end of the elastic member 912a abuts against one end of the first limiting member 911a, the other end of the first limiting member 911a abuts against the control button 913a, and the other end of the elastic member 912a abuts against the control button 913b.

During operation, the operator can simultaneously press the control button 913a and control button 913b inward with one hand, causing the first limiting member 911a and the first limiting member 911b to be unlocked synchronously.

To ensure more stable force transmission through the control button, the control button 913 is provided with two positioning structures on the inner side, namely: a first receiving groove 9131 positioned against the first limiting member in its own set, and a second receiving groove 9132 positioned against the elastic member in another set, thereby constraining the abutting ends of the first limiting member and the elastic member in fixed positions.

In order to stabilize the movement path of the first limiting member, two opposite sides of the first limiting member are provided with positioning posts, namely: a first positioning post 9112 extending into the corresponding first receiving groove 9131, and a second positioning post 9113 for the corresponding elastic member 912 to be fitted.

A hollow area 9012 is provided on the second housing 901, and a portion of the control button 913 is exposed and protrudes outward through the hollow area 9012. The edge of the control button 913 is provided with an anti-detachment rim 9133 inside the second housing 901, and the anti-detachment rim 9133 is constrained by the inner edge of the hollow area 9012.

The inner edge of the hollow area 9012 is ringed with a reinforcing seat 9013 extending into the second housing, the reinforcing seat 9013 is provided with a stabilizing groove 9014, and at least a portion of the anti-detachment rim 9133 extends into the stabilizing groove 9014, thereby securely fixing the control button 913 in the hollow area 9012 without any wobbling.

The first receiving groove 9131 and the second receiving groove 9132 are arranged in sequence along the axial direction. Since there is a gap defined between the second receiving groove 9132 and the connected end of the elastic member, the anti-detachment rim 9133 is provided with a lug 9134 adjacent to the second receiving groove 9132 for engaging the stabilizing groove 9014 on the side, thereby avoiding minor wobbling on the second receiving groove 9132 side of the control button 913.

Referring to FIG. 19, the proximal end of the extension sleeve 93 can be fixedly connected to the second handle 9 by snap-fitting or the like (the improved method is provided below), and the distal end of the extension sleeve 93 extends into the interior of the first handle 30 and is engageable with the second locking mechanism 92, thereby maintaining the second handle 9 in the second position.

Among them, a cooperating anti-detachment structure 932 is provided at the proximal end of the extension sleeve 93 inside the first handle 30, which defines the limit position of the extension sleeve 93 moving towards the proximal end, and prevents the extension sleeve from sliding excessively towards the proximal end or even detaching from the first handle. The anti-detachment structure 932 may be implemented by means of snap-fitting, plugging-in, overlapping, etc., and the improved method is also provided below.

Preferably, as shown in FIGS. 16 and 19, the anti-detachment structure 932 includes a protruding portion 9321 and a stopping portion 9322 that cooperate with each other. The protruding portion 9321 is located at the proximal end of the extension sleeve 93 and radially protrudes from the extension sleeve 93. The stopping portion 9322 is located inside the first handle 30. The stopping portion 9322 is distributed around the outer periphery of the protruding portion 9321 and acts as a barrier to further prevent the protruding portion 9321 from moving towards the proximal end.

In addition, the extension sleeve is provided with a convex rib 934, and the stopping portion 9322 is provided with a limiting groove that is engageable with the convex rib 934, which also serves to prevent the extension sleeve from rotating during the movement.

In some embodiments, as shown in FIG. 19, the second locking mechanism 92 includes a second limiting member 921 sleeved over the outer periphery of the extension sleeve, a spring 922 arranged between the first handle 30 and the second limiting member 921, and a releasing button 923 fixed to the second limiting member 921.

Regarding the cooperation structure between the extension sleeve and the second locking mechanism, the outer periphery of the extension sleeve 93 is provided with a second locking groove 931 that engages with the second limiting member 921, and the inner edge of the second limiting member 921 is provided with a second locking member 9211 that engages with the second locking groove 931. The second limiting member 921 has a locked state engaged with the extension sleeve 93 and a release state disengaged from the extension sleeve 93. The spring 922 drives the second limiting member 921 into the locked state, and the releasing button 923 drives the second limiting member 921 into the release state.

The connecting section 35 has a hollow structure for the tube to extend, the second limiting member 921 is located inside the connecting section 35, and the releasing button 923 extends through and is exposed from the side wall of the connecting section 35, so that the operator can directly apply force to the releasing button 923.

When the second handle 9 is in the first position, the second housing 901 shields the releasing button 923 to prevent accidental operation. When the second handle 9 is in the second position, the releasing button 923 is exposed from the second housing 901, and the operator can directly press the releasing button 923 to drive the second limiting member 921 into the release state.

The spring 922 and the releasing button 923 are located at two opposite sides of the second limiting member 921, so that the force applied by the operator can directly act on the second limiting member 921 through the releasing button 923, thereby enhancing the release efficiency.

Since the spring is prone to displacement during movement, the second limiting member 921 of the present application includes a guide post 9212 on the side opposite the releasing button 923. The spring 922 is sleeved over the guide post 9212 which also movably extends through the side wall of the connecting section 35, thereby guiding the axial direction of the spring to prevent it from deviating from the predetermined position.

During the movement of the outer sheath relative to the first handle, it is generally required to be locked when reaching the appropriate position. A common locking method involves constraining the driving sleeve. Moreover, when the sheath core assembly is quickly retracted, the relative fixation between the outer sheath and the first handle needs to be maintained to constrain the driving sleeve.

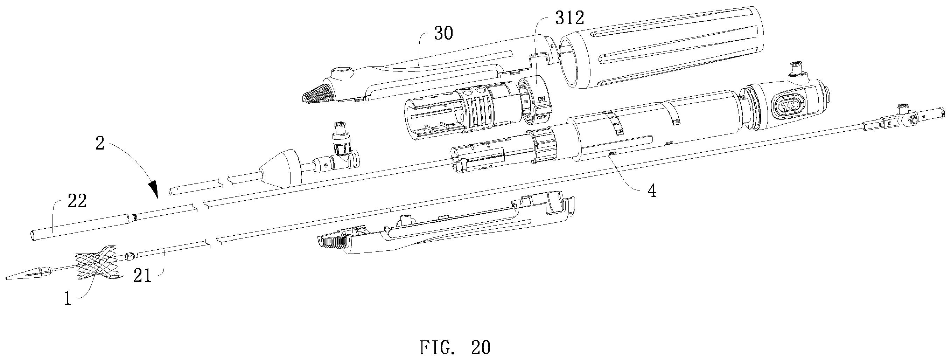

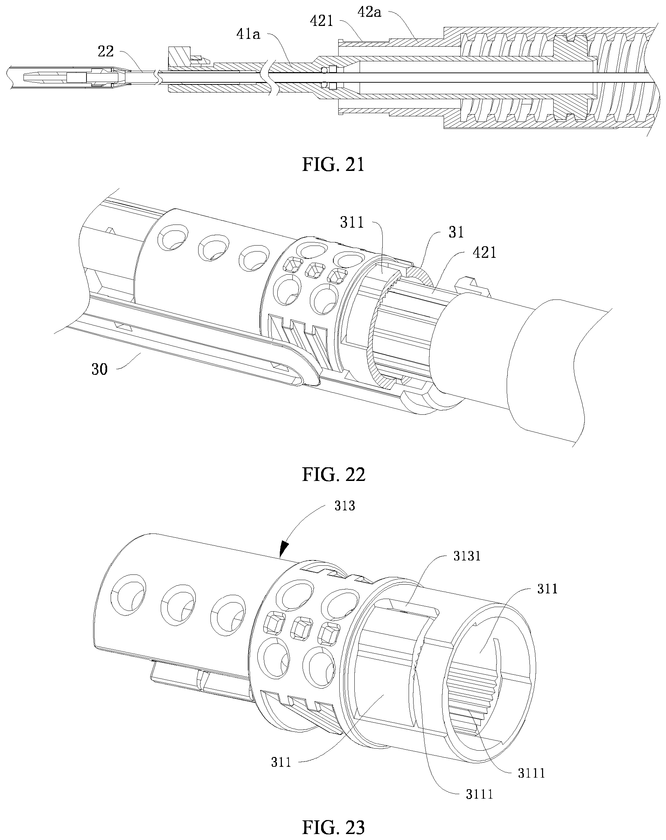

Referring to FIGS. 20 and 21, the control handle 3 in one embodiment of the present application includes a first handle 30 and multiple sets of driving mechanisms installed on the first handle 30, wherein one set of driving mechanisms 4 includes a movable base 41a and a driving sleeve 42a located on the outer periphery of the movable base 41a. The movable base 41a is axially slidably installed on the first handle 30 and configured to be connected to the corresponding tube (i.e., the outer sheath 22). The driving sleeve 42a is rotatably installed on the first handle 30, and the driving sleeve 42a and the movable base 41a are in a threaded transmission connection.

Referring to FIGS. 22 to 24, in order to facilitate the operator to lock the driving sleeve 42a and prevent accidental rotation, a portion of the first handle 30 forms a locking part 31 located at the outer periphery of the driving sleeve 42a, and at least a portion of the locking part 31 forms a radially deformable force-applying portion 311. The force-applying portion 311 has a locked state acting with the driving sleeve 42a to prevent the rotation of the driving sleeve 42a and a release state where it allows the driving sleeve to rotate.

The locking part 31 is sleeved over the outside of the driving sleeve 42a in coaxial alignment, with a fitting gap defined between the locking part 31 and the driving sleeve 42a, which serves as a deformation space for the force-applying portion 311. When subjected to radial force, the force-applying portion 311 will undergo radial deformation towards the central axis, such that the force-applying portion 311 is extendable into the fitting gap and comes into contact with the outer wall of the driving sleeve 42a, thereby entering a locked state where the rotation of the driving sleeve 42a is prevented. When the radial force is withdrawn, the force-applying portion 311 resets and exits the fitting gap, thus, the driving sleeve 42a is rotatable, and the force-applying portion enters the release state.

The force-applying portion 311 is an essential part for switching the locked state of the driving sleeve 42a. To ensure the locking effect of the driving sleeve 42a, the locking part 31 of the present application features a tubular structure 313, to improve the coaxiality with the driving sleeve 42a. A portion of the side wall of the cylindrical structure 313 also serves as the force-applying portion 311. The force-applying portion 311 is integrally formed with the tubular structure 313, which greatly simplifies the overall structure and reduces the assembly process. A hollow area 3131 for releasing deformation is provided between the force-applying portion 311 and the surrounding parts. The hollow area 3131 surrounds the periphery of the force-applying portion 311 and reserves space for the radial deformation of the force-applying portion 311. However, to ensure the overall strength of the tubular structure 313, the area of the hollow area 3131 should not be too large.

There are multiple force-applying portions 311 circumferentially spaced apart along the tubular structure 313, thereby increasing the force-applying area of the driving sleeve 42a, as well as providing more balanced force distribution on the driving sleeve 42a and enhancing the locking effect.

The circumferential span of the force-applying portion 311 along the tubular structure 313 is at least 1/6 circumference, which achieves both good locking effect and sufficient structural strength.

The inner side of the force-applying portion 311 is provided with first anti-slip teeth 3111 along the radial direction of the locking part 31. After the radial force deformation of the force-applying portion 311, the first anti-slip teeth 3111 can greatly increase the contact area, enhance the friction force, and thereby strengthen the locking effect compared to the force-applying portion of an inner smooth side.

Considering that it is inconvenient for an operator to directly apply a radial force to the force-applying portion, the present application provides an operating ring 312 rotatably sleeved over the outer periphery of the locking part 31, the inner wall of the operating ring 312 is provided with a working portion 3121 which is inward-protruding and engageable with the force-applying portion 311, and the working portion 3121 moves with the operating ring 312 to switch the states of the force-applying portion 311.

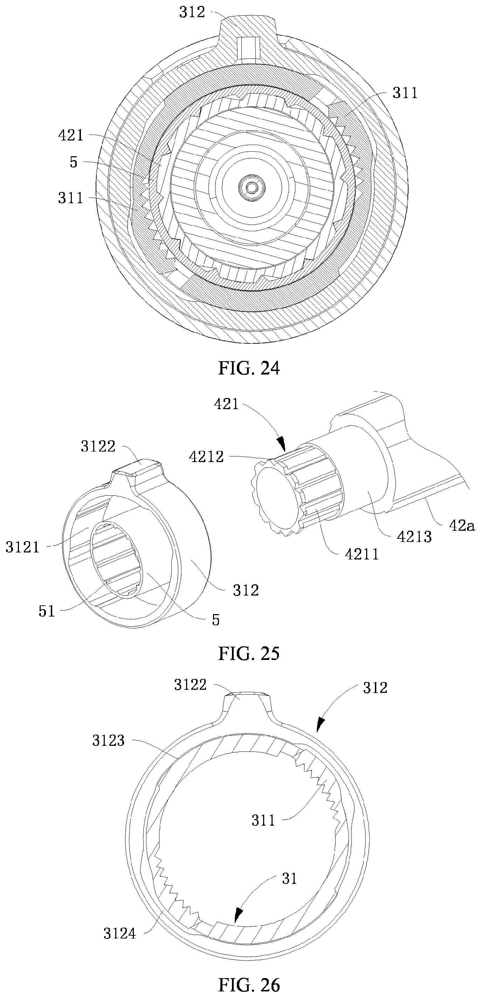

Referring to FIGS. 25 and 26, since the fitting gap is defined between the locking part 31 and the driving sleeve 42a, in case the force-applying portion 311 directly locks the driving sleeve 42a, it not only needs to pass through the fitting gap, but only the head area of the force-applying portion 311 can make contact with the driving sleeve 42a, and the contact area between the two components is always limited. Therefore, in the present application, a portion of the driving sleeve 42a is configured as an inserting section 421 that is engageable with the locking part 31, and an elastic locking ring 5 is sleeved over the outer periphery of the inserting section 421. When the driving sleeve 42a is in a locked state, the force-applying portion 311 abuts against the elastic locking ring 5 and prevents the rotation of the driving sleeve 42a. The elastic locking ring 5 is configured to fill the fitting gap between the locking part 31 and the driving sleeve 42a, allowing the force-applying portion 311 to press the inserting section 421 through the elastic locking ring 5 with only minimal radial deformation, which not only saves effort but also has an excellent locking effect.

The elastic locking ring 5 may be made of highly elastic material such as rubber, silicone, nylon, etc., which, through its deformation, not only prevents slipping but also provides a better haptic sensation and damping effect.

To further enhance the locking effect of the elastic locking ring 5 and the inserting section 421, a cooperating anti-slip structure is provided between the elastic locking ring 5 and the inserting section 421. The anti-slip structure includes at least one of the following:
second anti-slip teeth 51, distributed on the inner wall of the elastic locking ring 5; and
third anti-slip teeth 4211, distributed on the outer wall of the inserting section 421.

Both the second anti-slip teeth 51 and the third anti-slip teeth 4211 increase the friction between the elastic locking ring 5 and the inserting section 421, which further enhances the locking effect on the driving sleeve.

Preferably, the second anti-slip teeth 51 and the third anti-slip teeth 4211 engage with each other. As a result, the elastic locking ring 5 and the inserting section 421 hardly experience relative sliding during the abutment process, and the locking efficiency is very high, which further enhances the locking effect on the driving sleeve.

Regarding the axial constraint problem of the elastic locking ring 5, the distal end of the inserting section 421 is provided with a limiting flange 4212, and the proximal side is provided with a limiting ring 4213, thereby constraining the elastic locking ring 5 to the anti-slip structure of the inserting section 421, preventing the elastic locking ring 5 from detaching during operation and further assuring the locking effect.

To further enhance the operation effect and experience, as shown in FIG. 26, the operation ring 312 of the present application converts the rotational force of the operator into a radial force acting on the force-applying portion 311, thereby achieving more flexible control and a better operational experience. The outer wall of the operation ring 312 is provided with a radially protruding protrusion 3122, allowing the operator to apply the rotational force directly to the protrusion 3122. Compared to having the operator press directly on the outer wall of the operation ring 312, the protrusion 3122 allows for a more concentrated area of force application and saves more labor-saving for the operator.

The force-applying portion may include one or more units circumferentially spaced apart along the locking part 31. The inner wall of the operating ring 312 is alternately provided with concave areas 3124 and inner convex areas 3123 which serve as working portions, distributed in the circumferential direction. As shown in the figure corresponding to the configuration with two force-applying portions 311, the concave area 3124 in the inner wall structure is configured to accommodate the corresponding undeformed force-applying portion 311. Compared to the concave area 3124, the inner convex area 3123 is closer to the central axis in the radial direction, and the highest point of the inner convex area 3123 in the radial direction is lower than the highest point of the force-applying portion 311.

In the locked state, the outer side of the force-applying portion 311 abuts against the inner convex area 3123, so that the inner convex area 3123 compresses and deforms the force-applying portion 311 in the radial direction, and the force-applying portion 311 further prevents the rotation of the driving sleeve 42a.

In the release state, the outer side of the force-applying portion 311 corresponds to the concave area 3124, and the concave area 3124 reserves enough space for accommodating the force-applying portion 311, thereby allowing the force-applying portion 311 to remain in the reset state.

The concave area 3124 and the inner convex area 3123 are connected by a slope transition, so that the inner wall of the operating ring 312 constitutes a smooth curved surface as a whole, which ensures that the force-applying portion 311 will not be jammed or the like during the rotation of the operating ring 312.

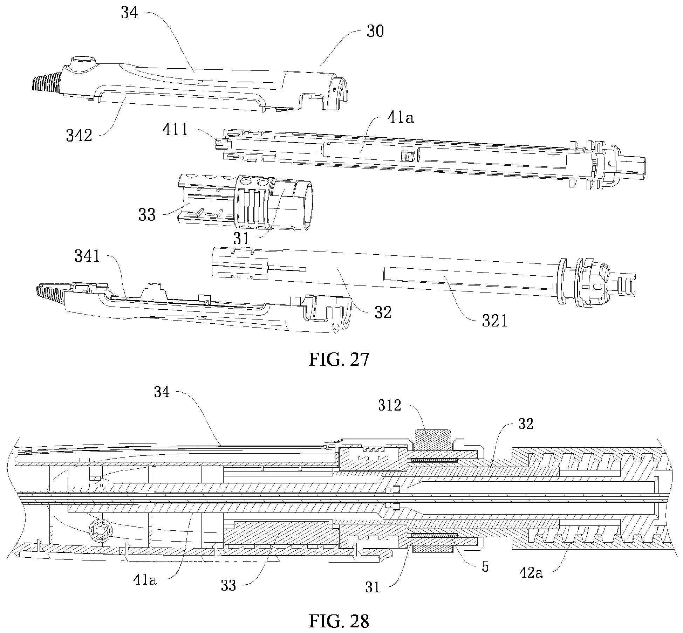

Referring to FIGS. 27 and 28, in order to make the first handle better cooperate with the movable base 41a and the driving sleeve 42a, the first handle 30 of the present application features a three-layer assembly structure which has a support body 32, an intermediate sleeve 33, and a first housing 34. The support body 32 mainly serves to fix and support various components. As shown in the figure, the movable base 41a is slidably mounted inside the support body 32, and the driving sleeve 42a is rotatably sleeved over the outer periphery of the support body 32.

The intermediate sleeve 33 serves to provide fixed support and constrained locking to the driving sleeve 42a. The distal end of the support body 32 in the figure is fixed to the intermediate sleeve 33 by a snap-fit structure known in the art, and the proximal end of the intermediate sleeve 33 is located at the outer periphery of the support body 32 and serves as a locking part 31, thereby reducing the parts of the control handle and simplifying the structure.

The first housing 34 is configured to cover the internal structure and to fixedly connect with other internal parts. In the figure, the first housing 34 encloses and is fixed to the outer periphery of the intermediate sleeve 33 through a snap-fit structure known in the art, with a first gap defined between the first housing 34 and the locking part 31 in the radial direction. The operating ring 312 is rotatably installed in the first gap and at least partially exposed from the first housing 34, making it easy for the operator to directly rotate the operation ring 312.

In addition, a second gap is provided between the support body 32 and the locking part 31 in the radial direction. The inserting section 421 on the distal end of the driving sleeve 42a extends into the second gap, further ensuring the assembly accuracy of the inserting section 421 and the locking part 31.

To intuitively reflect the operating progress of the interventional device on the control handle, the present application incorporates a visualization feature on the first housing 34. As shown in the figure, the first housing 34 is provided with an indicator window 341 extending along the axial direction, and the distal end of the movable base 41a is provided with a stroke indicator element 411. The stroke indicator element 411 is slidable along the indicator window 341, and the distal end and proximal end of the indicator window 341 respectively correspond to the limit positions of the movable base 41a sliding towards the distal end and the proximal end, such that the operator can directly observe the position of the stroke indicator element 411 to predict the operation progress of the interventional device, that is, achieving visualization of the operation progress.

Within the first housing, a shielding part 342 is provided in an area directly opposite to the indicator window 341, with a gap defined between the shielding part 342 and the indicator window 341 in the radial direction, which allows the stroke indicator element 411 to slide in the axial direction.

Some embodiments below further provide a method for releasing a prosthetic heart valve utilizing the delivery system described above, that is, an operation method of the delivery system of the above embodiments. The catheter assembly, the first handle, and the second handle mentioned below may be combined with the above embodiments.

In some embodiments, the delivery system includes a prosthetic heart valve (i.e., the aforementioned interventional device 1) having an expanded configuration and a collapsed configuration, and a delivery system for releasing the prosthetic heart valve. The prosthetic heart valve can enter and remain in the collapsed state from the expanded configuration through a loading device. The delivery system used includes a catheter assembly 2 and a control handle 3 connected to the proximal end of the catheter assembly 2, and the catheter assembly 2 includes an inner shaft assembly 21 and an outer sheath 22.

A radial gap defined between the distal ends of the inner shaft assembly 21 and the outer sheath 22 constitutes a loading zone for accommodating the interventional device. It can be understood that the loading zone is configured to accommodate a prosthetic heart valve in a collapsed state, and the prosthetic heart valve is connected to the loading zone. The connection method of those two components is provided below. The prosthetic heart valve is disengaged from the loading zone after being fully released.

The inner shaft assembly 21 and the outer sheath 22 are configured to be movable relative to each other, so that the loading zone is covered by or exposed from the outer sheath 22 (i.e. the prosthetic heart valve is covered or released), that is, the relative position state of those two tubes is reflected as the release state of the prosthetic heart valve.

The control handle 3 includes first and second handles 30, 9 that are slidably fitted together, and the first handle 30 is connected to the outer sheath 22. The second handle 9 is located on the proximal side of the first handle and configured to be connected to the inner shaft assembly. The second handle 9 has a first position where it combines with the first handle 30 (as shown in FIG. 3), and a second position where the second handle 9 slides proximally by a predetermined distance from the first position (as shown in FIG. 4). That is, the relative position change of the first handle and the second handle is also reflected in the relative position change of the inner shaft assembly 21 and the outer sheath 22. As shown in the figure, the first position corresponds to the outer sheath covering the loading zone, and the second position corresponds to the outer sheath exposing the loading zone.

The switching and maintaining of these two positions are realized by the first locking mechanism 91, the extension sleeve 93, and the second locking mechanism 92 arranged inside the control handle.

The first locking mechanism 91 is mounted to the second handle 9 and engageable with the proximal portion of the first handle 30 to maintain the first handle 30 in the first position. When the first locking mechanism 91 is unlocked, the second handle is slidable towards the proximal end relative to the first handle.

The extension sleeve is a connecting component between the two handles. The proximal end of the extension sleeve 93 is connected to the second handle 9, and the distal end of the extension sleeve 93 extends into the interior of the first handle 30.

The second locking mechanism 92 is installed on the first handle 30 and is engageable with the extension sleeve 93 to maintain the second handle 9 in the second position. The second locking mechanism 92 is concealed inside the second handle 9 in the first position, making it inaccessible for the operator to apply force directly. It is only after the second handle 9 slides towards the proximal end that the second locking mechanism 92 is exposed, allowing the operator to switch the operation.

Clinicians and other operators use the delivery system described above to place a prosthetic heart valve (hereinafter also referred to as interventional device 1) into a designated position in the body and release it. During the release process, the operator can determine the release timing of the prosthetic heart valve by observing indicators such as the position of the prosthetic heart valve in the body, its spatial posture, and its coordination with surrounding tissues in conjunction with the imaging equipment.

When releasing a prosthetic heart valve (i.e., interventional device 1), it includes:
maintaining the first handle 30 and the second handle 9 in the first position and delivering the prosthetic heart valve to the designated position, at which time the inner shaft assembly 21 and the outer sheath 22 are in the initial position and suitable for traversal in the body;
controlling the outer sheath 22 to slide towards the proximal end relative to the inner shaft assembly 21 via the first handle 30, exposing the loading zone to release the artificial heart valve;
unlocking the first locking mechanism 91, and controlling the inner shaft assembly 21 to slide towards the proximal end relative to the outer sheath 22 through the second handle 9, such that the outer sheath 22 covers the loading zone. As shown in FIG. 13 and FIG. 14, the second handle 9 is slidable towards the proximal end in the direction of arrow A, and the inner shaft assembly 21 can also be quickly retracted towards the proximal end in the direction of arrow B. The second locking mechanism 92 holds the first handle 30 and the second handle 9 in the second position; and
driving the catheter assembly 2 to move towards the proximal end away from the designated position via the first handle 30 and the second handle 9, and finally retracting the catheter assembly 2 from the body.

When controlling the outer sheath to slide toward the proximal end relative to the inner shaft assembly via the first handle, the state of the prosthetic heart valve is confirmed to be as expected upon reaching a predetermined release stage. Among them, when the prosthetic heart valve is released to the predetermined stage, the length of the prosthetic heart valve exposed from the outer sheath 22 is 55 % to 80 % of its axial total length. Preferably, the length of the prosthetic heart valve exposed from the outer sheath 22 is 60% to 70 % of its axial total length. It can be understood that the operator confirms that the position of the artificial heart valve in the body, its spatial orientation, and its coordination with surrounding tissues are as expected through imaging devices, etc.

If the state of the prosthetic heart valve does not meet expectations (i.e. the operator confirms that the position, spatial posture and other indicators of the prosthetic heart valve in the body are not as expected), the outer sheath is controlled to slide towards the distal end relative to the inner shaft assembly via the first handle, thereby re-enclosing the prosthetic heart valve, and then the state of the prosthetic heart valve is adjusted as needed.

After confirmation that it meets expectations, the outer sheath is further driven to fully expose the loading zone, thus completing the release of the prosthetic heart valve.

The technical features of the above embodiments can be arbitrarily combined, and not all possible combinations of the technical features of the above embodiments have been described for the sake of brevity of description. However, as long as there is no contradiction in the combination of these technical characteristics, such combination should be regarded as falling into the scope of this specification. When the technical features in different embodiments are shown in the same figure, it can be considered that the figure also discloses a combined embodiment of various embodiments involved.

The above-described embodiments only illustrate several embodiments of the present application, and the description thereof is specific and detail, but should not be construed as limiting the scope of the patent application. It should be noted that, for those of ordinary skill in the art, several modifications and improvements can be made without departing from the concept of the present application, all of which fall into the protection scope of the present application.

## Claims

1. A transcatheter delivery system, having opposite distal and proximal ends with an axial direction extending between the distal and proximal ends, the transcatheter delivery system comprising:
a catheter assembly comprising a plurality of tubes; and
a control handle connected to a proximal end of the catheter assembly, the catheter assembly comprising at least two tubes which are sleeved over one another, the control handle comprising a first handle and a plurality of sets of driving mechanisms, each set of driving mechanisms being in transmission cooperation with a corresponding tube of the catheter assembly;
wherein one set of the plurality sets of driving mechanisms comprises:
a driven member, mounted to the first handle and slidable along the axial direction, the driven member being used to connect to the corresponding tube; and
a driving member, rotatably mounted to the first handle, the driving member having internal threads and being located at an outer periphery of the driven member, and the driven member having transmission teeth that engage with the internal threads;
wherein the control handle further comprises one or more indicator members connected to the driving member and an elastic member connected to the driven member; and
wherein when the elastic member moves axially with the driven member to the position corresponding to the indicator member, the interaction between the elastic member and the indicator member provides a tactile feedback and/or a sound feedback.

2. The transcatheter delivery system according to claim 1, wherein the catheter assembly comprises an outer sheath connected to the driven member and an inner shaft assembly slidably fitted in the outer sheath, a radial gap defined between distal ends of the inner shaft assembly and the outer sheath constitutes a loading zone for accommodating an interventional device, and the inner shaft assembly and the outer sheath are configured to be movable relative to each other, so that the loading zone is covered by or exposed from the outer sheath; and
wherein the inner shaft assembly and the outer sheath have a predetermined relative position during relative movement, and in the predetermined relative position, the elastic member and the indicator member interact with each other to produce a response.

3. The transcatheter delivery system according to claim 2, wherein there are a plurality of relative positions which include at least a first relative position and a second relative position adjacent to each other in the axial direction;
wherein a total stroke of the inner shaft assembly relative to the outer sheath is M1; and
wherein a stroke of the outer sheath between the first relative position and the second relative position is M2, and M2 is at least 1/3 of M1.

4. The transcatheter delivery system according to claim 2, wherein the driving member is a driving sleeve and has a tubular structure, the indicator member is located on an inner side of the driving member, and the driven member is a movable base.

5. The transcatheter delivery system according to claim 4, wherein in the predetermined relative position, the indicator member produces a plurality of responses when rotation amplitude of the driving sleeve is less than or equal to one circle.

6. The transcatheter delivery system according to claim 2, wherein the indicator member is spaced apart from movement path of the transmission teeth; and
wherein the elastic member and the indicator member engage with each other when aligned, and deformation of the elastic member at least allows the indicator member to rotate with the driving member and override the elastic member.

7. The transcatheter delivery system according to claim 2, wherein the first handle at least comprises a support body in a tubular shape, the support body is provided with a guide groove extending axially, the driven member is slidably fitted in the guide groove, and the driving member is rotatably sleeved over an outer periphery of the support body; and
wherein the guide groove extends radially through the support body, and the transmission teeth are arranged on both sides of the driven member and extend out of the guide groove.

8. The transcatheter delivery system according to claim 2, wherein the driving member has a single-layer or double-layer structure, and the double-layer structure comprises:
a bushing, wherein receiving slots are formed on a side wall of the bushing, the indicator member is located within a corresponding receiving slot, and the internal threads are provided on an inner wall of the bushing, and
an outer housing fixedly sleeved over the bushing's periphery.

9. The transcatheter delivery system according to claim 8, wherein the indicator member and the bushing are of separate fixed structures.

10. The transcatheter delivery system according to claim 8, wherein an inner edge of the receiving slot is provided with a limiting step, and edges of the indicator member are fixedly seated on the limiting step.

11. The transcatheter delivery system according to claim 8, wherein extending direction of the indicator member is consistent with a thread angle of the internal threads.

12. The transcatheter delivery system according to claim 8, wherein along the axial direction, the one or more indicator members are arranged in a single set or more than two sets, with each set comprising one indicator member or a plurality of indicator members circumferentially spaced apart along the bushing.

13. The transcatheter delivery system according to claim 12, wherein circumferential extension length of a single indicator member along the bushing ranges from 1/4 to 1/2 of a circumference.

14. The transcatheter delivery system according to claim 12, wherein the indicator member at least comprises:
a first set of indicator members, wherein when the elastic member engages with the first set of indicator members, the interventional device is fully enclosed by the tube connected to the driven member; and
a second set of indicator members, wherein when the elastic member engages with the second set of indicator members, the interventional device is partially released by the tube connected to the driven member.

15. The transcatheter delivery system according to claim 12, wherein the indicator member comprises:
a main body, generally arc-shaped; and
protruding teeth distributed on an inner side of the main body, and there is a plurality of the protruding teeth spaced apart along an extension direction of the main body.

16. The transcatheter delivery system according to claim 15, wherein an outer periphery of the main body has a smooth structure, or an outer side of the main body is spaced with protruding placeholders.

17. The transcatheter delivery system according to claim 7, wherein the driven member comprises:
a base body, at least a portion thereof constituting a sliding part located inside the support body; and
the transmission teeth fixed to the sliding part, wherein the elastic member is connected to either the sliding part or the transmission teeth and extends out of the guide groove, or the elastic member is connected to the transmission teeth via an intermediate member, and the intermediate member is located outside the support body.

18. The transcatheter delivery system according to claim 17, wherein the intermediate member is provided with a positioning hole, through which the intermediate member is connected to the transmission teeth.

19. The transcatheter delivery system according to claim 17, wherein the elastic member is a bridge arm with two ends fixed to the intermediate member, and a central region of the bridge arm is engageable with the indicator member.

20. The transcatheter delivery system according to claim 19, wherein the central region of the bridge arm is provided with a protrusion that is engageable with the indicator member.

21. The transcatheter delivery system according to claim 19, wherein the intermediate member is provided with a deformation release opening arranged between the two ends of the bridge arm.

22. The transcatheter delivery system according to claim 17, wherein two radial sides of the intermediate member are respectively provided with the elastic member.

23. The transcatheter delivery system according to any one of claims 2 to 22, wherein comprising another set of driving mechanisms, which comprises:
a second handle, located on the proximal side of the first handle and configured to be connected to the proximal end of the inner shaft assembly, the second handle having a first position where it engages with the first handle and a second position slid proximally by a predetermined distance; and
at least one set of locking mechanisms arranged between the first handle and the second handle to maintain their relative positions.

24. The transcatheter delivery system according to any one of claims 4 to 22, wherein a portion of the first handle forms a locking part located at the outer periphery of the driving sleeve, and at least a portion of the locking part forms a radially deformable force-applying portion, and the force-applying portion has a locked state where it interacts with the driving sleeve to prevent its rotation, and a release state where it allows the driving sleeve to rotate; and
an operating ring is rotatably sleeved over the periphery of the locking part, and an inner wall of the operating ring is provided with a working portion which is radially inward-protruding and engageable with the force-applying portion, and the working portion moves together with the operating ring to switch states of the force-applying portion.

25. A transcatheter delivery system, having opposite distal and proximal ends with an axial direction extending between the distal and proximal ends, the transcatheter delivery system comprising:
a catheter assembly; and
a control handle connected to a proximal end of the catheter assembly, the control handle comprising a first handle and a plurality of sets of driving mechanisms, each set of driving mechanisms being in transmission cooperation with a corresponding tube of the catheter assembly;
wherein one set of the plurality sets of driving mechanisms comprises a second handle located at a proximal end of the first handle and configured to connect with the corresponding tube, and the second handle has a first position where the second handle engages with the first handle and a second position where the second handle slides proximally by a predetermined distance from the first position;
wherein a first locking mechanism and a second locking mechanism are provided between the first handle and the second handle;
wherein the first locking mechanism is configured to retain the first handle in the first position, and comprises:
a first limiting member, having an annular structure and radially slidably mounted on the second handle, an inner edge of the annular structure serving as a locking member that cooperates with the first handle; and
a state component, acting on at least one radial side of the first limiting member for retaining the first limiting member in a locked state engaged with the first handle, or switching it to a release state disengaged from the first handle;
wherein the second locking mechanism is configured to retain the second handle in the second position.

26. A transcatheter delivery system, having opposite distal and proximal ends with an axial direction extending between the distal and proximal ends, the transcatheter delivery system comprising a catheter assembly and a control handle connected to the proximal end of the catheter assembly, the catheter assembly comprising an inner shaft assembly and an outer sheath, a radial gap defined between the distal ends of the inner shaft assembly and the outer sheath constituting a loading zone for accommodating an interventional device, the inner shaft assembly and the outer sheath being configured to be movable relative to each other so that the loading zone is covered by or exposed from the outer sheath;
the control handle comprising a first handle and a plurality of sets of driving mechanisms, each set of driving mechanisms being in transmission connection with a corresponding tube of the catheter assembly; and one set of driving mechanisms comprising a driving sleeve, rotatably mounted to the first handle;
wherein a portion of the first handle forms a locking part located at an outer periphery of the driving sleeve, and at least a portion of the locking part forms a radially deformable force-applying portion, and the force-applying portion has a locked state interacting with the driving sleeve to prevent its rotation and a release state where it allows the driving sleeve to rotate; and
wherein an operating ring is rotatably sleeved over an outer periphery of the locking part, and an inner wall of the operating ring is provided with a working portion which is radially inward-protruding and engageable with the force-applying portion, and the working portion moves with the operating ring to switch states of the force-applying portion.

27. A method for releasing a prosthetic heart valve by a delivery system,
the prosthetic heart valve having an expanded configuration and a collapsed configuration;
the delivery system having opposite distal and proximal ends with an axial direction extending therebetween, the delivery system comprising a catheter assembly and a control handle connected to a proximal end of the catheter assembly, the catheter assembly comprising an inner shaft assembly and an outer sheath, a radial gap defined between distal ends of the inner shaft assembly and the outer sheath serving as a loading zone for accommodating an interventional device, the inner shaft assembly and the outer sheath being configured to move relative to each other to allow the loading zone to be covered by or exposed from the outer sheath;
the prosthetic heart valve being releasably connected to the loading zone;
the control handle comprising a first handle and a second handle slidably fitted together;
the first handle being configured to connect the outer sheath;
the second handle being located on a proximal end of the first handle and configured to connect the inner shaft assembly; and the second handle having a first position where the second handle engages with the first handle and a second position where the second handle slides proximally by a predetermined distance from the first position;
a first locking mechanism being mounted to the second handle and engageable with the proximal end of the first handle to maintain the first handle in the first position;
an extension sleeve with its proximal end of the extension sleeve connected to the second handle and its distal end extending into an interior of the first handle;
a second locking mechanism being mounted to the first handle and being configured to maintain the second handle in the second position; and
wherein the method comprises steps of:
maintaining the first handle and the second handle in the first position and delivering the prosthetic heart valve to a designated position;
controlling the outer sheath to slide towards the proximal end relative to the inner shaft assembly via the first handle, exposing the loading zone to release the prosthetic heart valve;
unlocking the first locking mechanism and controlling the inner shaft assembly to slide towards the proximal end relative to the outer sheath via the second handle, so that the outer sheath covers the loading zone, and at this time, the second locking mechanism maintains the first handle and the second handle in the second position; and
driving the catheter assembly to move toward the proximal end away from the designated position via the first handle and the second handle.
